(19) **Europäisches Patentamt / European Patent Office / Office européen des brevets**

(11) **EP 4 772 835 A1**

(12) **EUROPEAN PATENT APPLICATION**

(43) Date of publication:
08.07.2026 Bulletin 2026/28

(21) Application number: 25150221.7

(22) Date of filing: 03.01.2025

(51) International Patent Classification (IPC):
*G01C 21/36* *(2006.01)*

(52) Cooperative Patent Classification (CPC):
**G01C 21/3697; G01C 21/3629; G01C 21/3641; G01C 21/3655**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: HERE Global B.V.
**5611 ZT Eindhoven (NL)**

(72) Inventors:
- **Beaurepaire, Jerome**
  **92400 Nantes (FR)**
- **Slater, Olaf**
  **65842 Schwalbach am Taunus (DE)**
- **Falla Cepeda, David**
  **10115 Berlin (DE)**

(74) Representative: **Sariot, Eray**
**HERE Global BV**
**Parnassusweg 715**
**1077 DG Amsterdam (NL)**

(54) **METHOD, APPARATUS AND COMPUTER PROGRAM PRODUCT FOR ROUTE-DEPENDENT CONTENT GENERATION**

(57)     Example embodiments describe a computer-implemented method, an apparatus, and a computer program product for generating personalized content for users traversing a route. The method may obtain a route and its content attention profile, which assigns an estimated content attention level to each road link based on user attention levels corresponding to various road link features. A control prompt may be generated based on this profile and a first content data file containing text, audio, or video data. The control prompt may instruct adjusting the detail level of the content. A generative artificial intelligence component may process the first content data file and control prompt to generate a second content data file. The second content data file may be presented on a user device. This method may provide an adaptive entertainment experience for users along their route, potentially enhancing engagement and enjoyment during travel.

Fig. 1

EP 4 772 835 A1

**Description**

**FIELD**

The following disclosure relates to a method, apparatus, and computer program product for generating content for a user traversing a route based on characteristics or properties of the route.

**BACKGROUND**

As maps for the road network become richer in both terms of coverage and detail, technologies aimed to enhance the vehicle experience offer ever more features beyond "from A to B" navigation. For instance, the increasing advance of technologies in vehicles, such as advanced driver-assistance systems (ADAS) and even Autonomous Driving (AD) systems, has also opened the door to new challenges and opportunities in terms of passenger entertainment and information delivery. Existing methods for providing content to passengers during travel are often limited by their inflexibility and lack of personalization.

Further advances in Artificial Intelligence (AI) and Machine Learning (ML) continue to enable new applications, for example by analyzing user behavior and generating personalized content in near real-time. Generative AI (GenAI) is nowadays capable of generating text, audio, still images and video on command.

Despite such recent developments, content consumption on route, in particular aboard vehicles, remains a fairly disconnected experience from the traveled route. For example, playback of different media types still follows a lineal path regardless of the current situation of the user on board the vehicle. Solutions for improving the content consumption experience while traveling may increase the understanding, safety and overall enjoyment of the journey.

**SUMMARY OF SOME EXEMPLARY EMBODIMENTS**

According to a first exemplary aspect, a computer-implemented method is disclosed, the method comprising:

- obtaining a route for a user based on an origin location and a destination location, the route comprising a plurality of road links;

  generating a content attention profile associated with the route, via a route analysis component, by assigning an estimated content attention level to each of the road links of the plurality of road links based on a correlation of one or more road link feature types of the respective road link with a user attention level corresponding to the one or more of road link feature types;

  obtaining a first content data file, wherein the first content data file comprises one or more of text, audio and video data;

  generating a control prompt based on the first content data file and the content attention profile, wherein the control prompt comprises instructions for adjusting a level of detail of at least a portion of the content of the first content data file;

  providing, to a generative artificial intelligence, GenAI, component, the first content data file and the control prompt;

  obtaining, from the GenAI component, a second content data file, wherein the second content data file is generated based on the first content data file and the control prompt; and

  causing presentation of the second content data file on a device associated with the user during traversal of the route.

The user attention level according to the first exemplary aspect may be, in some embodiments, determined based on: a) an alertness level of a human subject, the alertness level being determined via sensor observations made onboard a vehicle while the vehicle is traversing a road link having road link features corresponding to the one or more road link feature types, and b) a user profile. In yet other embodiments, the user profile may be one of a driver profile, a passenger profile, a manually controlled vehicle driver profile, a semi-autonomous vehicle driver/passenger profile, or an autonomous vehicle passenger profile.

In some embodiments of the method according to the first exemplary aspect, the sensor observations may comprise gaze tracking, heart rate, breathing rate, speech monitoring, vehicle control input, or a combination thereof. In yet other embodiments, the sensor observations may be based on the user and/or one or more human subjects other than the user.

In some embodiments of the method according to the first exemplary aspect, the correlation between the one or more road link feature types and the user attention level is learned using a supervised machine learning technique.

In yet other embodiments of the method according to the first exemplary aspect, the content attention profile may be further generated based on a collective user attention level for a plurality of users of the device.

In some embodiments of the method according to the first exemplary aspect, the at least one or more road link feature types may comprise at least one of a road geometry, a lane count, a functional classification, a speed limit, a road surface type, a road surface hazard indication, an autonomous driving suitability indication, or a geographical area designation.

In yet other embodiments of the method according to the first exemplary aspect the at least one or more road link feature types may further comprise at least one of a traffic level, a weather condition, a pedestrian density level, a noise level, a variable speed limit, a construction zone, an autonomous driving availability indication, or an accident hazard indication.

In some embodiments of the method according to the first exemplary aspect, the origin location may be a current device location, a predicted origin location, or a user input origin location, and the destination location may be a predicted destination location, or a user input destination location.

In some embodiments according to the first exemplary aspect, the method may further comprise associating the route with the second content data file; and, storing the second content data file for presentation during a future route traversal.

In some embodiments according to the first exemplary aspect, the method may further comprise providing, to the GenAI component, an estimated route traversal time to adjust the duration of the second content data file.

In some embodiments, the control prompt may further comprise instructions for providing the second content data file of a media type different to the first content data file.

In some embodiments according to the first exemplary aspect, the method may further comprise:

- requesting user input based on the content of the second content data file;
- obtaining the user input from the user; and,
- adjusting the correlation of the at least one or more road link feature types with the user attention level based on the obtained user input.

According to the first exemplary aspect of the invention, an apparatus is disclosed, configured to perform and/or control, or comprising respective means for performing and/or controlling the method according to the first exemplary aspect.

Generally, the means of the apparatus of the first exemplary aspect can be implemented in hardware and/or software. They may comprise for instance at least one processor for executing computer program code for performing the required functions, at least one memory storing the program code, or both. Alternatively, they could comprise for instance circuitry that is designed to implement the required functions, for instance implemented in a chipset or a chip, like an integrated circuit. In general, the means may comprise for instance one or more processing means or processors.

More specifically, according to the first exemplary aspect, an apparatus is disclosed, comprising at least one processor and at least one memory including computer program code, the at least one memory and the computer program code configured to, with the at least one processor, cause an apparatus at least to perform and/or to control the method according to the first exemplary aspect.

The apparatus may be at least one server performing the method according to the first exemplary aspect. The server may for instance be or comprise a server of a map provider or distributor. The at least one server may also be realized by multiple servers or a distributed architecture and may for instance be or comprise a distributed server, a network edge server, or a cloud server. The server may also comprise a database (e.g. a map database) or be in communication with an (e.g. external) database or a database server. As will be explained in more detail below, a part of the server may for instance be a software and/or hardware component thereof, such as a functional component, a server component, module, a circuitry, a chip, a microprocessor or the like.

In some embodiments, the method according to the first exemplary aspect, the method may be performed locally on a mobile device, such as a computing unit of a vehicle (e.g. infotainment unit) or a mobile phone/smartphone. In such embodiments, the above-disclosed apparatus may be a module or a component of the mobile device, for example a chip. The disclosed apparatus according to any aspect of the invention may comprise only the disclosed components, for instance processor, memory, or may further comprise one or more additional components. For example, the apparatus may be a "System-on-a-Chip" or SoC, comprising the aforementioned components. The apparatus may also be implemented such that the, the processor, memory or one or more additional components are shared by other functions of the mobile device.

In yet other embodiments, the method according to the first exemplary aspect may be performed by a system comprising a server and a mobile device (e.g. server and mobile device as described above), coupled over a communication network. For example, method steps oriented towards obtaining sensor data, user interactions or content playback may be performed by the mobile device, while method steps oriented towards route analysis, learning preferences or generating content may be performed on the server, taking advantage of more powerful processing resources. It should be understood that several combinations are possible, and embodiments of the system may be configured to perform the tasks associated with the method via different components according to different implementation choices.

According to the first exemplary aspect, a computer program product is also provided. The computer program product comprises instructions which, when the program is executed by a computer, cause the computer to carry out any computer-implemented method disclosed herein.

In some embodiments, the computer program product may be stored on computer-readable storage medium, in particular a tangible and/or non-transitory medium. The computer readable storage medium could for example be a disk or a memory or the like. The computer program product could be stored in the computer readable storage medium in the form of instructions encoding the computer-readable storage medium. The computer readable storage medium may be intended for taking part in the operation of a device, like an internal or external memory module, for instance a Read-Only Memory (ROM) or hard disk of a computer, or be intended for distribution of the program, like an optical disc.

The features and example embodiments described above may equally pertain to the different aspects.

It is to be understood that the presentation in this section is merely by way of examples and non-limiting.

Other features will become apparent from the following detailed description considered in conjunction with the accompanying drawings. It is to be understood, however, that the drawings are designed solely for purposes of illustration and not as a definition of the limits, for which reference should be made to the appended claims. It should be further understood that the drawings are not drawn to scale and that they are merely intended to conceptually illustrate the structures and procedures described herein.

## BRIEF DESCRIPTION OF THE DRAWINGS

The embodiments of the invention are illustrated by way of example, and not by way of limitation, in the figures of the accompanying drawings:

**Fig. 1** shows a diagram of a system arrangement in accordance with some example embodiments.

**Fig. 1a** depicts a route comprising an origin, a destination and route segments in accordance with some example embodiments.

**Fig. 1b** depicts the route overlaid on a map in accordance with some example embodiments.

**Fig. 2** shows an exemplary embodiment of an apparatus in accordance with some example embodiments.

**Fig. 3a-c** shows components utilized in embodiments in accordance with some example embodiments.

**Fig. 4a** depicts a route comprised of multiple route segments and corresponding route features in accordance with some example embodiments.

**Fig. 4b-e** provides exemplary graphs of attention levels corresponding to route segments in accordance with some example embodiments.

**Fig. 5** depicts a flowchart of a computer-implemented method in accordance with example embodiments.

**Figs. 6a-6b** depict an example operational scenario in accordance with example embodiments.

**Fig. 7** shows examples of data storage hardware in accordance with some example embodiments.

## DETAILED DESCRIPTION OF SOME EXAMPLE EMBODIMENTS

The following description shall be understood to complement and be read together with the description as provided in the above summary section of this description. Examples of a computer-implemented method, apparatus, and computer program for logistics robot deployment are disclosed. In the following description, for the purposes of explanation, numerous specific details are set forth in order to provide a thorough understanding of the embodiments of the invention. It is apparent, however, to one skilled in the art that the embodiments of the invention may be practiced without these specific details or with an equivalent arrangement. In other instances, well-known structures and devices are shown in block diagram form in order to avoid unnecessarily obscuring the embodiments of the invention.

Entertainment on board vehicles has evolved hand in hand with other automotive technologies. At the dawn of the automotive industry, the sheer complexity of operating the first vehicles required constant driver attention to simply maintain the engine running, while the actual task of driving was a daunting coordination exercise to direct the vehicle away from hazards and in the right direction. The lack of paved roads and the general public's tenuous understanding of the rules of the road only increased the complexity even more, which most probably left any driver without any available attention for enjoying any sort of entertainment. Passengers would also be similarly overwhelmed by the noise and roughness of the ride alone, such that even a normal conversation presented a challenge.

Fast-forward in time and vehicle manufacturers started to incorporate more user-friendly drivetrains, smoother steering, suspension systems, cabins with some form of sound absorption, as well as radio receivers. Nowadays, digital systems are capable of obtaining sensor readings from the environment surrounding the vehicle, from the environment inside the vehicle, and even from the occupants (driver or passengers) of the vehicle. Coupled with digital road network maps, technologies such as Advanced Driver Assistance Systems (ADAS) are now able to control multiple aspects of the driving experience, increasing efficiency, safety, comfort and reducing the cognitive load of the driver. For example, drivetrains can now take into account features such as inclination of the current stretch of road to select the right gear in the transmission, adjust fuel flow rates, adjust electric motor / internal combustion engine balance (on hybrid drivetrain models); headlamps can adjust to road curvature or inbound light from other vehicles; steering wheel speed/sensitivity may equally respond to road curvature and a speed profile for the road; cameras may identify signs such as speed limits, "stop", "merge", "do not enter", "one-way" and relay the information to the occupants; etc.

Inside the vehicle, multiple sensor systems may nowadays be deployed to monitor the occupants' current state of alertness, such as camera(s) for gaze tracking, microphones capturing sounds made by occupants (e.g. speech monitoring), steering wheel grip sensors, vehicle control input sensors, vital signs (pulse, breathing, temperature, blood pressure, oxygenation, etc.) sensors. Attaining a level of alertness from these systems allows some ADAS applications to raise warnings when the driver becomes distracted or to adjust warning mechanisms if e.g. important safety information appears not to be acknowledged by the occupants while traversing a portion of a route. For example, while some roads may be recognized or labeled (e.g. in a digital road network map) as apt for Autonomous Driving, different levels of (Semi-) Autonomous Driving vehicles (e.g. SAE Automation levels 2, 3 and 4) may still require that the driver stays awake and ready to take the wheel in different situations. Such cases may use at least a subset of the aforementioned occupant monitoring sensors to determine if the person in the driver's seat is indeed attentive (enough) to take over.

Entertaining systems on board vehicles have progressed enormously as well, some even with immersive, "home theater"-like sound and video capabilities. The availability of content, once limited to radio broadcasts, cassettes, "8-track" tapes, CDs, etc., has reached a practical infinite with the introduction of streaming services coupled with the ubiquitous presence of high-speed mobile internet services e.g. over cellular networks. Vehicle occupants can now enjoy multiple forms of audio-visual content while traversing a route. Even drivers having handed over the driving task to a (semi-) Autonomous Driving vehicle can also enjoy a full variety of content, while also enjoying predominantly audible content during manual driving.

Even with all the aforementioned advances, content consumption aboard vehicles remains a substantially linear content playback affair, in the best of cases with an estimated travel time matching the duration of some piece of pre-recorded content. Even in such cases the content is manually selected by the user in view of the estimated travel time. The Inventors have recognized that the connection between the properties of a route and a vehicle occupant's capability to attentively consume a piece of content is still completely ignored. Example embodiments disclosed herein aim to provide solutions that, at least, provide optimized content during routes to be traveled or being traveled.

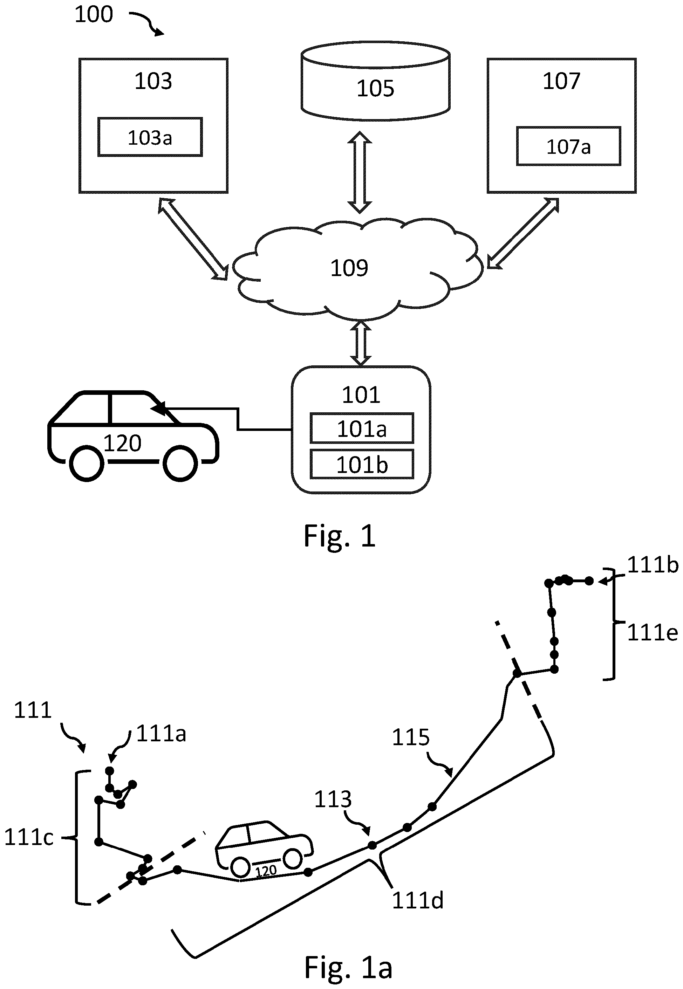

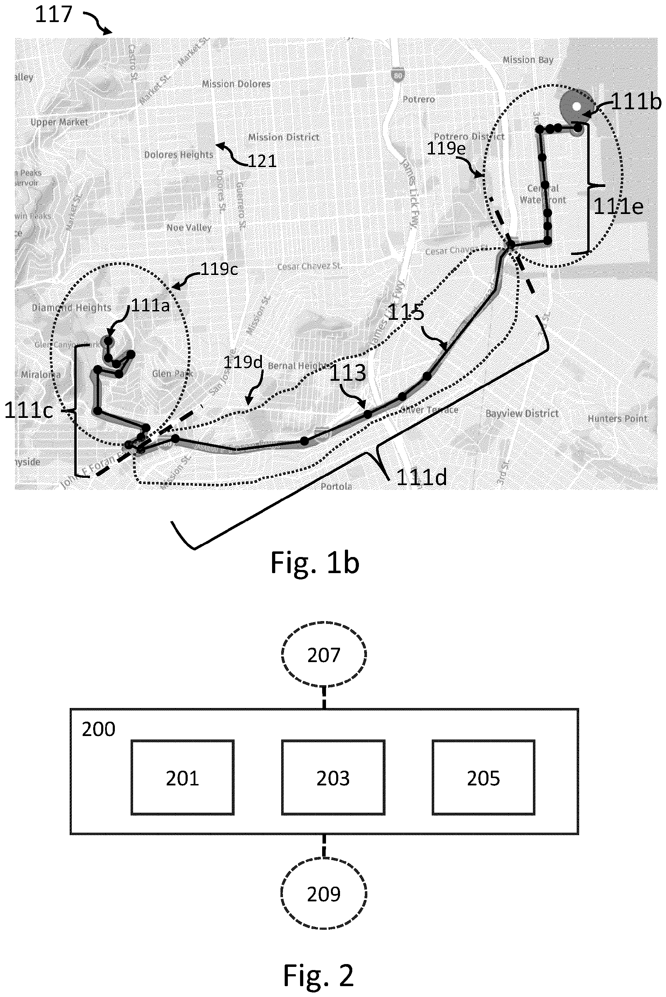

Attention is now turned to Fig. 1, depicting a system arrangement 100, comprising a mobile device 101, a content delivery platform 103, a map database 105, and a Location-Based Services (LBS) platform 107. The aforementioned components are interconnected via a communications network 109. The mobile device 101 is further depicted comprising a user monitoring component 101a, and a user interface module 101b. The content delivery platform 103 is further depicted comprising a Generative Artificial Intelligence component, GenAI component 103a. The LBS platform 107 may also comprise a route analysis component 107a. Alternative configurations are envisioned, for example, the map database 105 being implemented within any of the mobile device 101, the GenAI component 103a or the LBS platform 107. Similarly, the GenAI component 103a, the user monitoring component 101a, the route analysis component 107a may be implemented as stand-alone services, or as part of other components of system arrangement 100. As the communication network 109 interconnects the various components/modules, multiple other configurations in different embodiments are also possible. The system arrangement of Fig. 1 may be implemented utilizing components described in connection with previously mentioned apparatuses.

The mobile device 101, also referred to as "Device" or "User's Device", may be a portable computing device designed for personal use, such as a smartphone, a mounted infotainment unit of a vehicle 120, or a similar device. Mobile device 101 may be embodied by an apparatus akin to apparatus 200. Mobile device 101 may run an operating system such as Android, Android Automotive, QNX, Windows, iOS, Linux or the like. The mobile device 101 may be equipped with a processor that can handle various tasks, such as GNSS data processing, AI computations, and multimedia playback. Examples of processors include Qualcomm Snapdragon, Apple A-series, or Intel Core, m-series. Furthermore, mobile device 101 may be equipped with sufficient memory (RAM) to run the operating system, applications, and various other background processes. The mobile device may also include non-volatile storage, such as a solid-state drive (SSD), for storing data, apps, and content files. Mobile Device 101 may further comprise wireless connectivity options such as Wi-Fi 6, Bluetooth 5.0, NFC, and cellular connectivity (e.g., 4G LTE or 5G), as well as wired (e.g. CAN adapter) for communication with the vehicle's onboard systems and external networks.

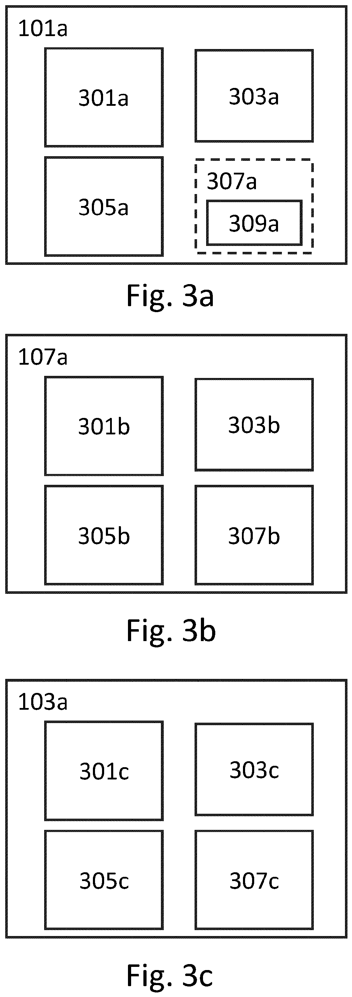

Mobile device 101 is further depicted in Fig. 1 to comprise a user monitoring component 101a and a user interface 101b. The user monitoring component 101a may further include or be in communication with at least one sensor of the mobile device 101 or of the vehicle 120, such as the sensors 209 described below in connection with the apparatus 200. Examples of sensor packages oriented towards driving alertness monitoring, may be Harman Automotive's Ready Care, Tobii Autosense, Seeing Machines FOVIO, or similar solutions. In some embodiments, the user monitoring component 101a may be also implemented as a stand-alone component, for example as another component of the vehicle 120, as a remote service on cloud computing infrastructure, with access to sensor observations pertaining to the occupants of the vehicle 120, e.g. over communication network 109. The user monitoring component 101a will be further described in connection with Fig. 3a.

Furthermore, mobile device 101 is depicted to comprise a user interface 101b. The user interface 101b may be akin to the user interface 207 described in connection with apparatus 200 of Fig. 2. It is to be understood that a user of the mobile device may be referred to as a human subject, a driver, a passenger, or an occupant of a vehicle, depending on the situation. In general, a user in the context of example embodiments may be a human subject from which a) sensor observations may be obtained and b) may consume content (either by actively selecting it or passively by being in the vehicle while being played) via the mobile device 101. The user interface 101 may receive input from a user (e.g. a vehicle occupant) pertaining to route calculation (e.g. origin, destination, waypoints, route preferences, etc.), media content (e.g. content selection, playback control, sound/image adjustment, etc.), or control of other mobile device functions (e.g. power functions, application selection, device/vehicle settings, etc.). In addition, the user interface 101b may be capable of providing the user with information pertaining to the route (e.g. route between origin and destination overlaid on a map, traffic levels or events on route, etc.), information about available media content (e.g. a media library, a selected file, duration of media, etc.), other vehicle system information (e.g. HVAC settings, vehicle health status, vehicle settings, ADAS warnings, connectivity status, etc.), as well as being capable of media playback itself. The user interface 101b may utilize visual, auditory, tactile (e.g. Braille), haptic channels to receive or convey information to the user.

Further depicted on Fig. 1 is the content delivery platform 103, which may be embodied by an apparatus akin to apparatus 200. The content delivery platform 103 may be capable of allowing a user to select a piece of media content (media content data file), for presentation on e.g. the user interface 101b of mobile device 101. The content delivery platform 103 is depicted comprising a Generative Artificial Intelligence, GenAI, component 103a. The GenAI component 103a may also be implemented in some embodiments as a stand-alone component, in communication with the route analysis component 107a, the mobile device 101 and content delivery platform 103 via the communication network 109. Alternatively, the content delivery platform 103 or the GenAI component 103a may be implemented in other embodiments as part of the mobile device 101 or as part of the LBS platform 107, also in communication with other aforementioned components via communication network 109. The GenAI component 103a may be capable of receiving a media content data file, a content attention profile, a control prompt, and outputting a subsequent media content data file based on the received media content data file and the content attention profile. The GenAI component 103a will be described in more detail in connection with Fig. 3b.

Returning to the map database, the map database 105 may include node data, road segment data or link data, point of interest (POI) data, or the like. The map database 105 may also include cartographic data, routing data, and/or maneuvering data. According to some example embodiments, the road segment data records may be links or segments representing roads, streets, or paths, as may be used in calculating a route or recorded route information for determination of one or more personalized routes. The node data may be end-points corresponding to the respective links or segments of road segment data. The road link data and the node data may represent a road network, such as used by vehicles, cars, trucks, buses, motorcycles, and/or other entities. Optionally, the map database 105 may contain path segment and node data records or other data that may represent pedestrian paths or areas in addition to or instead of the vehicle road record data, for example. The road links / road segments and nodes can be associated with attributes of different types describing features, such as geographic coordinates, road geometry information, lane count, functional classification (e.g. freeway, highway, collector, arterial, local etc., coded as e.g. FC 1-5), speed limit, road surface type, road surface hazard indication, autonomous driving suitability indication, geographical area designation, and other navigation related attributes, as well as POIs, such as fueling stations, hotels, restaurants, museums, stadiums, offices, auto repair shops, buildings, building footprints, stores, parks, etc.. The map database 105 can include data about the POIs and their respective locations in the POI records. The map database 105 may include data about places, such as cities, towns, or other communities, and other geographic features such as bodies of water, mountain ranges, etc. Such place or feature data can be part of the POI data or can be associated with POIs or POI data records (such as a data point used for displaying or representing a position of e.g. a business, a public office, a venue, a town, a city, etc.). In addition, the map database 105 can include event data (e.g., traffic incidents, construction activities, scheduled events, unscheduled events, etc.) associated with the POI data records or other records of the map database 105.

The map database 105 may be maintained by a content provider e.g., a map developer. By way of example, the map developer can collect geographic data to generate and enhance the map database 105. There can be different ways used by the map developer to collect data. These ways can include obtaining data from other sources, such as municipalities or respective geographic authorities. In addition, the map developer can employ field personnel to travel by vehicle along roads throughout the geographic region to observe features and/or record information about them, for example. Also, remote sensing, such as aerial or satellite photography, can be used to generate map geometries directly or through machine learning.

The map database 105 may be a master map database stored in a format that facilitates updating, maintenance, and development. For example, the master map database or data in the master map database can be modeled in a Map Object Model (MOM) format, such as for development or production purposes. The Oracle spatial format or development/production database can be compiled into a delivery format, such as a geographic data files (GDF), GeoJSON, or NDS format. The data in the production and/or delivery formats can be compiled or further compiled to form geographic database products or databases, which can be used in end user navigation devices or systems. The map database 105 may be queried via a user interface, an API, or the like. A query to the map database 105 may, for example, specify a location in various formats (coordinates, addresses, road link ID, map tile ID, areas, polygons, regions, POI ID, etc.) and may further allow for searching within a distance around a location (e.g. location + search radius in meters, search corridors around a road link or sequence of road links, along a route, within a map tile, or the like).

For example, geographic data may be compiled (such as into a platform specification format (PSF) format) to organize and/or configure the data for performing navigation-related functions and/or services, such as route calculation, route guidance, map display, speed calculation, distance and travel time functions, and other functions, by a mobile device or a location-based service, such as, respectively, mobile device 101 or LBS Platform 107, for example.

The LBS Platform 107 may provide further geolocated information. In some embodiments, the map database 105 may be co-located or be made accessible through the LBS Platform 107. Static and dynamic information may be obtained by querying the LBS Platform 107 by providing a location indication (e.g. coordinate pair, road link ID, map tile ID, etc., similar to queries discussed in connection with the map database 105) and optionally an information type and time parameter (e.g. hh:mm, dd/mm/yyyy or similar) via an API. A reply may then contain the available information. The mobile device 101 or the route analysis component 107a, may request information from the LBS platform 107. The mobile device 101 or the route analysis component 107a may access location-based services to obtain information relevant to route planning and route segment analysis tasks. Such location-based services may include, but are not limited to:

- Weather services: providing current and forecasted weather conditions, including temperature, precipitation, wind speed, and other meteorological data;
- Traffic services: providing real-time traffic congestion patterns, road closures, variable speed limits, and construction information;
- Hazards services: providing information on hazards such as accidents, roadwork, or natural disasters that may affect navigation;
- Mobility density/ pedestrian traffic services: providing information on pedestrian density, flow rates, and movement patterns to inform route planning and obstacle avoidance;
- Noise level information: providing information of noise levels caused by construction, heavy traffic, public events, airports and the like that may affect vehicle occupant attention;
- Autonomous driving availability indications: providing information for availability of autonomous driving functions. Semi/autonomous driving systems, in particular SAE levels 2,3 and 4, may insist that a plurality of road link conditions are met before enabling autonomous driving modes, such as a traffic speed range, a number of lanes, a lack of reported accidents, appropriate road paint marking, lighting, weather and visibility conditions, or the like.

In Fig. 1, the route analysis component 107a is depicted as part of the LBS platform 107. Certain embodiments may be implemented by co-locating the route analysis component 107a with the LBS platform 107, as this may, for example, allow for faster access to computed routes, fresher data for performing route analysis tasks, access to more powerful computing resources, and the like. Still, as previously mentioned, the route analysis component 107a may be implemented in other embodiments as a stand-alone component (i.e. removed from the LBS platform 107), as a component of the mobile device 101, or of any other service, utilizing the communication network 109 to interact with other components, in embodiments where e.g. different constraints regarding data security/privacy, allocation of processing resources or hardware limitations, may influence implementation choices. Both LBS Platform 107 and route analysis component 107a may be embodied by an apparatus akin to apparatus 200. Route analysis component 107a will be further described below in connection with Fig 3c.

Fig.1a depicts an exemplary route 111 comprising an origin 111a and a destination 111b, to be traversed or being traversed by a vehicle 120 comprising mobile device 101. The route 111 is depicted to comprise route segments 111c, 111d, and 111e. The different route segments 111c-111e represent portions of the route with different characteristics, some characteristics derived from road link properties (of road links corresponding to the route segment, such as road link 115), other derived from properties of the area adjacent to the route segment, such as areas 119c-119e. It is to be understood that a route may be divided into more or less route segments and that many other different characteristics may be attributed to the route segment(s) than in the present example. In example embodiments, the route may be divided into route segments comprising individual road links. In some example embodiments, route segments may also comprise a fragment of a road link.

In general, road links, such as road link 115, represent a stretch of road between two junctions in a map, for example in map database 105. The junctions may be represented by nodes, such as node 113. Road link features, represented as road link attributes in, e.g. map database 105, may comprise an attribute type (e.g. roadGeometry, laneCount, speedLimit, etc.) and a corresponding attribute value (e.g. "curvy", "3", "30km/h", etc.). The road link feature may be associated with the full length of the road link or with a portion thereof, for example by specifying a validity range for the feature (e.g. speedLimit=30km/h; range=[35m,150m]) along the road link measured from one node in the direction of travel. Road link feature information may be also obtained from other services, such as LBS Platform 107. Road link features may correspond to road link feature types, which may include road geometry, a lane count, a functional classification, a speed limit, a road surface type, a road surface hazard indication, an autonomous driving suitability indication or a geographical area designation. Furthermore, road link features may be obtained pertaining to a traffic level, a pedestrian density level, a noise level, a variable speed limit, a construction zone, an autonomous driving availability indication, or an accident hazard indication.

Fig. 1b is a further depiction of route 111, overlaid on map area 117. The example corresponds to the San Francisco, CA area, where a variety of road features are present on a single route. It should be understood that multiple other areas around the world may exhibit the same or different road feature combinations, for which example embodiments herein disclosed remain similarly applicable. The map area 117 includes multiple other mapped road links, such as road link 121 (a portion of Dolores St.), which may not form part of route 111, but may still be documented on map database 105. Map database 105 may further comprise feature information for different areas, such as areas 119c, 119d, and 119e, which may be associated with any road link 115 of the route. Alternatively or in addition, a service such as LBS platform 107 may further provide information associated with any road link 115, 121, or associated with any area 117, 119c-e. As a road link may be located within an area or a portion thereof, features or information pertaining to the area may be inherited by said road link. For example, information regarding weather conditions, noise levels, air pollution, geographical (e.g. rural/industrial/urban/mixed) area designations, pedestrian density, etc. may be available for an area, such that road links contained within the area may inherit the same information as a further road feature by virtue of their location.

Proceeding with Fig. 2, an example embodiment of a mobile device 101 as an apparatus is provided. The apparatus, such as that shown in Fig.2, may be specifically configured in accordance with an example embodiment of the present disclosure for performing a method in accordance with the first exemplary aspect. The apparatus may include or otherwise be in communication with a processor 201, a memory device 203, a communication interface 205, and a user interface 207. In some embodiments, the processor (and/or co-processors or any other processing circuitry assisting or otherwise associated with the processor) may be in communication with the memory device via a bus for passing information among components of the apparatus. The memory device may be non-transitory and may include, for example, one or more volatile and/or non-volatile memories. In other words, for example, the memory device may be an electronic storage device (for example, a computer readable storage medium) comprising gates configured to store data (for example, bits) that may be retrievable by a machine (for example, a computing device like the processor 201). The memory device may be configured to store information, data, content, applications, instructions, or the like, for enabling the apparatus to carry out various functions in accordance with an example embodiment of the present invention. For example, the memory device could be configured to buffer input data for processing by the processor. Additionally or alternatively, the memory device could be configured to store instructions for execution by the processor.

The processor 201 may be embodied in a number of different ways. For example, the processor may be embodied as one or more of various hardware processing means such as a coprocessor, a microprocessor, a controller, a digital signal processor (DSP), a processing element with or without an accompanying DSP, or various other processing circuitry including integrated circuits such as, for example, an ASIC (application specific integrated circuit), an FPGA (field programmable gate array), a microcontroller unit (MCU), a hardware accelerator, a special-purpose computer chip, or the like. As such, in some embodiments, the processor may include one or more processing cores configured to perform independently. A multi-core processor may enable multiprocessing within a single physical package. Additionally or alternatively, the processor may include one or more processors configured in tandem via the bus to enable independent execution of instructions, pipelining and/or multithreading.

In an example embodiment, the processor 201 may be configured to execute instructions stored in the memory device 203 or otherwise accessible to the processor. Alternatively or additionally, the processor may be configured to execute hard coded functionality. As such, whether configured by hardware or software methods, or by a combination thereof, the processor may represent an entity (for example, physically embodied in circuitry) capable of performing operations according to an embodiment of the present invention while configured accordingly. Thus, for example, when the processor is embodied as an ASIC, FPGA or the like, the processor may be specifically configured hardware for conducting the operations described herein. Alternatively, as another example, when the processor is embodied as an executor of software instructions, the instructions may specifically configure the processor to perform the algorithms and/or operations described herein when the instructions are executed. However, in some cases, the processor may be a processor specific device (for example, a mobile terminal or a fixed computing device) configured to employ an embodiment of the present invention by further configuration of the processor by instructions for performing the algorithms and/or operations described herein. The processor may include, among other things, a clock, an arithmetic logic unit (ALU) and logic gates configured to support operation of the processor.

The apparatus 200 of an example embodiment may also include a communication interface 205 that may be any means such as a device or circuitry embodied in either hardware or a combination of hardware and software that is configured to receive and/or transmit data to/from a communications device in communication with the apparatus, such as to facilitate communications with other entities such as content delivery platform 103, map database 105, LBS platform 107 or the like via communication network 109. In this regard, the communication interface may include, for example, an antenna (or multiple antennae) and supporting hardware and/or software for enabling communications with a wireless communication network. Additionally or alternatively, the communication interface may include the circuitry for interacting with the antenna(s) to cause transmission of signals via the antenna(s) or to handle receipt of signals received via the antenna(s). In some environments, the communication interface may alternatively or also support wired communication. As such, for example, the communication interface may include a communication modem and/or other hardware and/or software for supporting communication via cable, digital subscriber line (DSL), universal serial bus (USB) or other mechanisms.

The apparatus 200 may also include a user interface 207 that may in turn be in communication with the processor 201 to provide output to the user and, in some embodiments, to receive an indication of a user input. As such, the user interface may include a display and, in some embodiments, may also include a keyboard, a mouse, a joystick, a touch screen, touch areas, soft keys, one or more microphones, a plurality of speakers, or other input/output mechanisms. In one embodiment, the processor may comprise user interface circuitry configured to control at least some functions of one or more user interface elements such as one or more displays and, in some embodiments, a plurality of speakers, a ringer, one or more microphones and/or the like. The processor and/or user interface circuitry comprising the processor may be configured to control one or more functions of one or more user interface elements through computer program instructions (for example, software and/or firmware) stored on a memory accessible to the processor (for example, memory device 203, and/or the like). Alternatively, or in addition, the user interface 207 may be implemented as a service for other apparatuses or systems, and may be made accessible via communication interface 205, such as a web-page interface, remote desktop/console, or the like.

The apparatus 200 of some embodiments may be integrated with or otherwise onboard the vehicle 120 whereby the apparatus 200 may be equipped with or in communication with (e.g., via communications interface 205) one or more sensors 209, such as a Global Navigation Satellite System (GNSS) sensor (e.g., GPS, Galileo, GLONASS, etc.), accelerometer, image sensor, LiDAR, radar sensor, ultrasonic sensor, infrared sensor, inertial measurement unit (IMU), gyroscope, magnetic field sensor, etc. Any of the sensors may be used to sense information regarding the location, movement, positioning, or orientation of the apparatus for use in identifying a location of the apparatus 200. In some embodiments, the apparatus 200 may derive information regarding location, movement, position, or orientation of the apparatus 200 based on communication signals perceived by the communications interface 205 such as through signal triangulation or signal fingerprinting, for example. In some embodiments, the apparatus may combine both sensor information and communication signals to derive a location of the apparatus 200.

The apparatus may further comprise sensors 209 suitable for performing observations of a human subject, such gaze tracking, heart rate, breathing rate, speech monitoring or vehicle control input. It should be noted that vehicle control input, while often used to control different vehicle systems at the driver's will, may also be considered a human observation as it measures the activity level of the driver while controlling the vehicle. Sensors 209 may also comprise one or more of a visual spectrum camera, an infrared IR camera, a radar-based sensor for contactless measurement of vital signs, such as heart rate, heart rate variability, or breathing rate, a microphone, heart rate electrodes (e.g. on the steering wheel for heart rate measurement), oxygenation meters, pressure sensors (e.g. for measuring accelerator/brake input), steering angle sensors (e.g. for measuring steering wheel input), or other sensors capable of obtaining the aforementioned observations of a human subject(s)/occupant(s) of the vehicle.

In some embodiments, for example when the mobile device 101 is a portable navigation device, a smartphone or another device not permanently mounted to the vehicle 120, the mobile device 101 may embody an apparatus 200, comprising at least one of a camera and a microphone as sensors 209. When positioned in an appropriate location within the vehicle (e.g. with unobstructed view of the occupant's/s' face), the camera may be able to e.g. track eye gaze, occupant's/s' movement or other activity in the vehicle 120's cabin. Some state-of-the-art approaches are even capable now of inferring a heart rate from minute variations on facial skin tone, invisible to the human eye but detectable via camera. Similarly, the microphone may be able to e.g. perceive a noise level, sporadic noises, conversation, etc., in the vehicle 120's cabin. Other sensor observations made by other sensors may be performed by either the apparatus 200 (to the extent that the sensors are available to it) or by connected devices (e.g. via communication interface 205) of the apparatus 200, such as wearables (e.g. smartwatches) which can reliably measure vital signs (heart rate, heart rate variability, oxygenation, breathing rate, temperature, etc.) or hand movements (e.g. movements associated with steering, interaction with vehicle systems such as infotainment, HVAC, seat adjustments, etc.) which may indicate different levels of activity.

In some other embodiments, for example when the mobile device 101 is integrated with the vehicle 120, as a navigation system, infotainment unit, or the like, the mobile device 101 may embody an apparatus 200, in connection with sensors 209 deployed in different locations within the vehicle's cabin. Sensors 209 may be placed such that reliable sensor observations of the occupant(s). For example, visible spectrum cameras or IR cameras may be mounted such that an unobstructed view of the occupant's/s' eyeballs, microphones may be mounted away from air vents that introduce noise, radar-based sensors may be mounted such that heart and breathing patterns can be collected without interference, electrodes may be mounted on the steering wheel to measure heart pulse signals from the driver, or vehicle control input sensors, already used for controlling braking, acceleration, steering or other vehicle functions may relay observations of interactions with these functions as sensor data corresponding to the driver. Sensors 209 may be connected via the communication interface 205 to the apparatus, implemented using a Controller Area Network CAN, or other suitable connection. Other sensor observations, for example from wearable devices as described above, may communicate with the apparatus 205 via other implementations of the communication interface 205, such as wireless local area networks (e.g. Wi-Fi, Bluetooth, etc.).

In yet other embodiments, the apparatus 200 may be remotely located, away from the vehicle 120. A server, computing cluster, cloud computing infrastructure, or the like may embody components of the apparatus, such as the processor 201, the memory device 203 or the communication interface 205. In such case, the user interface 107 and the sensor(s) 209 may be part of a mobile device akin to mobile device 101, may be on board a vehicle 120, and may communicate with the apparatus 200 via the communication interface 205, resulting in a "thin client" configuration.

Proceeding with Fig 3a, the user monitoring component 101a is depicted in further detail. User monitoring component 101a includes a sensor observation reception module 301a, a user profile module 303a, a user attention estimation module 305a, and a profile adjustment module 307a. It should be apparent that equivalent implementations which achieve similar results are possible, for example utilizing more or less modules or by implementing different functionalities in the different modules.

Sensor observation reception module 301 may be enabled to receive sensor observations pertaining to the user(s) of mobile device 101. Sensor observations may comprise gaze tracking, heart rate, breathing rate, speech monitoring, vehicle control input, (body) temperature or the like. As an example, gaze tracking sensor observations may be quantified using the PERCLOS metric (percentage of eyelid closure over the pupil over time), eye blink speed metric, gaze direction (e.g. in deg or off-the-road/on-the-road), head orientation, or the like. Similarly, heart rate or breathing rate (measured in beats/breaths per minute) may be tracked over time to detect changes; temperature (e.g. in °C) may similarly be tracked; speech may be monitored utilizing metrics such as words per minute, or other audible events (e.g. instances of laughter, shouting, crying, snoring, etc.). Vehicle control inputs may be tracked to detect e.g. sudden/brisk control input, lagging control input, or number of event interactions with comfort features (e.g. infotainment, HVAC, etc.).

User profile module 303a may contain profile information associated with different users. The users may be users of a vehicle. The vehicle, e.g. vehicle 120, may be manually controlled, semi-autonomously controlled, or autonomously controlled. The user profile may be a driver profile or a passenger profile. For example, the user profile may be one of a driver profile, a passenger profile, a manually controlled vehicle driver profile, a semi-autonomous vehicle driver/passenger profile, or an autonomous vehicle passenger profile. In other embodiments, user profiles may be included for similar or additional user types. For example, users may correspond to "smartphone user while driving", "smartphone user while passenger", "smartphone user - child", "driver", "passenger", "child passenger", "driver-turned-passenger in AV mode" (semi-autonomous), "passenger of AV" (fully autonomous), or the like. Further user profiles may be based on age, gender, psychological profile, content preferences, etc. could also be defined. In addition, individual user profiles (e.g. Taylor's profile, Charly's profile, etc.) may also be defined, for instance by manual entry, customization of a previously existing profile or the like. In yet other embodiments, a "pedestrian user" profile may be utilized for e.g. a pedestrian traversing a route without a vehicle.

A user profile in the context of example embodiments may contain reference ranges for gaze tracking, heart rate, breathing rate, speech monitoring, vehicle control input, (body) temperature or the like, that correspond to ranges of user alertness. User alertness may be expressed, for example, utilizing e.g. a normalized metric $AL_{sensor}(t) \in [0,1]$, where 0 indicates a user in sleeping state and 1 a user in completely alert state at a given time t. It should be apparent that other metrics may achieve similar results.

User attention levels may be determined based on an alertness level of a human subject, the alertness level being determined via sensor observations made onboard a vehicle while the vehicle is traversing a road link having road link features corresponding to the one or more road link feature types. A user attention level may be considered as a user's attentiveness to consume content. Metrics such as the aforementioned user alertness $AL_{sensor}$ in connection with a user profile may serve as a proxy for user attention levels. For example, sensor observations determining that a subject, in the role of a driver, has their eyes predominantly on the road, is breathing normally and has a constant, normal heart rate, may indicate that the subject is in a "receptive" alertness state. It may be reasonably assumed that the user attention level may also correspond to "receptive". Similarly, in the role of a driver, a heightened breathing/heart rate (e.g. due to a complex driving situation, such as a curvy road, an intersection, overtake, a highway exit, etc.) may indicate that the driver, while still "receptive", may have a lower user attention level for content consumption. In contrast, observations of a subject in the role of passenger can also result in similar sensor observations than for a driver, however the user attention level may still be e.g. higher than the driver, as the passenger is not tasked with driving. Sensor observations may comprise gaze tracking, heart rate, breathing rate, speech monitoring, vehicle control input, or a combination thereof. Multiple studies have been performed that associate sensor observation metrics of the different sensors with a subject's attention state while driving, sitting, resting, etc., determining that said associations vary for different human subject categories, similar to the aforementioned user categories. Such studies are conducted with multiple human subjects to allow for robust attention level estimations. As similar or the same sensor observations are utilized in studies of user attention level and user alertness, usable correlations emerge, further supporting the use of user alertness as proxy for user attention levels. Accordingly, a database accessible to the user profile module 303a with statistical sensor observation data collected for the different user profiles during attention study experiments may be then utilized as a baseline dataset to determine a level of attention corresponding to a human subject. The user profile module 303a may provide, based on classification (e.g. subject corresponds to "driver" category) or identification (e.g. user is "Taylor") of the user of the mobile device 101, a corresponding user profile. User classification or user identification may be performed by methods such as user input, biometric identification, computer vision, voice recognition, etc.

User attention level estimation module 305a may be configured to match incoming user sensor observations from sensor observation reception module 301a to a corresponding level of attention A(t). For the sake of simplicity, exemplary embodiments will be described in relation to gaze tracking sensor observations, although it should be apparent that the aforementioned sensor observations may be handled in a similar manner. For example, a gaze tracking metric $GT=[gd,ebs]$, where $gd$ is a ratio of gaze direction between time off-the-road/time on-the-road, and $ebs$ a normalized eye blink speed, may be utilized. The user attention level estimation module 305a may obtain a current user observation gaze metric $GT_{t1}$ via the sensor observation reception module 301a, obtain a corresponding user profile from the user profile module 303a, and provide an attention level An based on a reference gaze metric $GT_{ref}$ of the corresponding user profile. For example, $GT_{t1}=[0.3, 0.2]$ may correspond to a user being *"alert"* (e.g. looking at the road often, low blink rate). The attention level An, estimated via user alertness $AL_{sensor}(t_1)$, may yield a numeric result, may be expressed on a scale of 1-10 (or equivalent), ranges (e.g. *low, low-med, normal, med-high, high*), attributes (e.g. *unresponsive, distracted, concentrated, alert, receptive, stressed*), or similar.

Analogously, multiple additional sensor observations, such as heart rate ($HR$), breathing rate ($BR$), speech monitoring ($SpM$), vehicle control input ($VI$), (body) temperature ($BT$) or the like may be combined towards calculating an attention level estimation, provided corresponding reference human subject sensor observation metrics are available for the different user profiles. A resulting alertness level $AL_{sensor(t)}$ may be calculated as $AL_{sensor}(t)=aHR(t)+bBR(t)+cSpM(t)+dVI(t)+eBT(t)$, wherein $a,b,c,d,e$ are weighting coefficients selected according to the contribution each sensor observation type may provide, or based on implementation choices. The alertness level in connection with a user profile may be then utilized as a proxy metric for an attention level. It should be noted that the above example is non-limiting and provides only an example combination of multiple sensor observation combinations possible to estimate a user attention level. Furthermore, metrics derived from sensor observations may serve as data for estimating user attention, given that attention is highly individual, and no methods are known to fully and accurately anticipate the state of mind of a human subject.

In some embodiments, the alertness metric $AL_{sensor}(t)$ may come from a user alertness sensor package (e.g. Harman Automotive's Ready Care, Tobii Autosense, Seeing Machines FOVIO, or similar solutions) and may further provide the sensor observations used for calculation of A(t) (e.g. $HR(t); BR(t); SpM(t); VI(t); BT(t)$, etc.)

The user monitoring component 101a may further comprise, in some embodiments, a profile adjustment module 307a. The profile adjustment module 307a may further obtain information about a user's current alertness level $AL_{sensor}(t)$ and associate it with road features a road link being traversed. It is submitted that a user's attention level may vary according to the characteristics of different portions of the route and current user role while traversing a route, such that an attention level factor linked to road features, e.g. $A_{road}$, and a user profile factor $P_{user}$, may be applied to derive an adjusted attention level $A_{adj}$. The factors $A_{road}$, and $A_{adj}$ may be normalized (e.g. range [0,1]) and variable, for instance in dependence of road link features (e.g. $A_{road}(roadFeatureTuple)$), or on contextual information relevant for the user

profile, e.g. $P_{user}$(contextData). $P_{user}$ may also be a fixed value for a user profile category (e.g. 0,2 for a driver, 0,9 for a passenger, 0,7 for a child passenger, 0,8 for a driver while vehicle in AD mode, etc.). For example, a low-traffic, single lane, straight rural road, in the morning when the user is rested, may cause few distractions to a user. Conversely, a busy (e.g. high speed but dense traffic), 4-lane highway, or urban areas with high pedestrian density, after working hours, may cause user attention levels to be lower as e.g. more distractions are available. It should be noted that a vehicle's driver may be focused on the task of driving and navigating the vehicle, such that said user's alertness level $AL_{sensor}$ is e.g. "alert", but as currently driving, the driver user may still be considered as less attentive for content consumption in connection with example embodiments (e.g. leading to a lower value for $P_{user}$). In contrast, a passenger (or the same driver while the vehicle is on AD mode) may be considered attentive without accounting for attention for the task of driving, such that the estimated attention level for the user substantially equal to the detected alertness level $AL_{sensor}$. Furthermore, a passenger's attention may be also influenced by road link features of a route, for example, multiple lanes, higher speeds, traffic levels, etc. being distracting to a passenger, albeit to a different degree than to a driver, still yielding an attention level factor linked to road features $A_{road}$. Accordingly, associations between attention levels and road features may be made for separate user profile categories (driver, adult passenger, child-passenger, etc.), including individual/personal user profiles (e.g. "Taylor's" profile, "Charly's" profile). These associations may, in turn, provide for an adjusted attention level $A_{Adj}$ based on the features of the road link, e.g.

$$A_{adj} = AL_{sensor} \times \alpha(\, P_{user}(contextData) \times A_{road}(roadFeatureTuple)) \,,$$

with $\alpha$ as an optional adjustment coefficient as may be required by a specific implementation (here $\alpha = 1$). $A_{adj}$ may be considered a resulting attention level $A$ (or $A(t)$).

By map-matching a current location (as provided by e.g. GNSS location sensors), a corresponding road link stored in, for example map database 105, can be identified, and associated road link features can be retrieved. For example, road link attributes pertaining to features like road geometry, lane count, functional classification, speed limit, road surface type, road surface hazard indication, autonomous driving suitability indication, or geographical area designation may be associated with an attention level $A_{road}$. Similarly, road link attributes pertaining to traffic level, weather condition, pedestrian density level, noise level, variable speed limit, construction zone, autonomous driving availability indication, an accident hazard indication may be retrieved, for example from LBS Platform 107 and may be associated with an attention level $A_{road}$. Furthermore, contextual information data such as time of day, weekday, workday/day off, season, other occupants in vehicle, etc. may also be collected and associated with an attention level $A_{road}$. A ground truth repository may be formed once sufficient road link attribute instances (e.g. >10000) have been collected (optionally, with corresponding contextual information), such that patterns in the instances are discernible in connection with different attention levels. A classifier 309a implemented as part of the profile adjustment module 307a may be trained to determine which road link feature combinations correlate to which levels of attention. Classifier 309a may be implemented utilizing supervised machine learning methods, such as a Support Vector Machine, Random Forest, Bayes classifier, or the like. In such implementations, the input variables may correspond to road features or in addition, optionally, to contextual information, while the output variables correspond to an estimated attention level value $A \in [0,1]$, estimated discrete attention level ranges, e.g. (*low, low-med, normal, med-high, high*) or estimated attention level categories (e.g. *unresponsive, distracted, concentrated, alert, receptive, stressed*). Notably, the classifier 309a need not be trained on board a specific mobile device 101, but may also be trained on other infrastructure, for instance cloud infrastructure, which may collect multiple attention observations from multiple mobile devices akin to mobile device 101 (e.g. via crowdsourcing), thus increasing the available ground truth samples which may lead to improved attention level estimations. Instances of classifier 309a may be made available to other components of system 101, by means of copying or connectivity via communication network 109.

Attention is now turned towards Fig. 3b, which depicts route analysis component 107a, comprising a route reception module 301b, a route segment analysis module 303b and a content attention profile output module 305b, as well as a classifier 307b. Route reception module 301b is configured to receive a route to be traversed by the user, in general on board a vehicle 120. The route may be calculated by a service akin to LBS platform 107, by a routing application of mobile device 101, or the like. The route may be based on an origin location and a destination location, such as an origin location 111a or destination location 111b. The origin location and the destination location may be determined via user input (e.g. interaction with user interface 101b) or may be inferred from a current vehicle location (as origin location) and a predicted destination location, for example a destination location determined based on user habits, historical data, calendar entries or the like. Utilizing a predicted destination location may offer the advantage of a more flexible allocation of computing resources to perform content generation (e.g. hours before departure), instead of performing content generation contemporaneously with user-initiated route calculation. An output content data file may, in such cases, be generated for a predicted route, and be stored until the route is traversed in the future. The route, for example route 111, may comprise a sequence of road links (such as road link 115) to be traversed from the origin location to the destination location. Each road link of the route may have corresponding road features stored as road attributes on a map database, such as map database 105 or otherwise accessible via another service, such as LBS platform 107. In addition, route properties, such as an estimated traversal time or an estimated time of arrival may be provided along with the route. Alternatively, an estimated traversal time may be calculated or otherwise retrieved by the route reception module 301b or another component of the system 100.

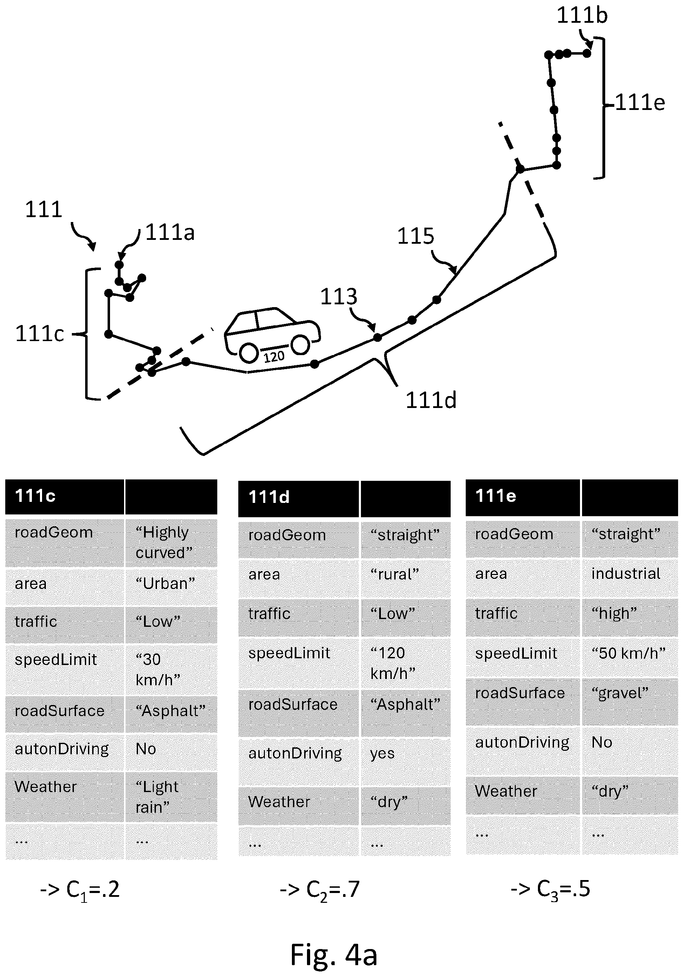

The received route, here example route 111, may be provided to the route segment analysis module 303b. The route segment analysis module 303b may be configured to segment the route into subsets of sequential links of the route 111, e.g. route segment 111c, 111d, 111e. It should be understood that multiple different segmentations are envisioned and that the above selection of three segments is solely for ease of explanation. Routes may be segmented into groups of links, individual road links, or even portions thereof. Segmentation may be done based on estimated time to traverse (e.g. first X minutes of route, second X minutes of route, third X minutes of route, etc.), functional classification changes (e.g. groups of links in sequence before/after a functional classification change happens), area designation changes, etc. For each route segment 111c, 111d, 111e, corresponding road link attributes are obtained from map database 105 or LBS platform 107. In case of multiple road links in a route segment, road attributes may be summarized into a list of e.g. most frequently occurring/predominant road attributes for each corresponding route segment 111c, 111d, or 111e. Fig 4a depicts an exemplary road feature summary for route 111.

The route segment analysis module 303b may be further configured to obtain a user profile from the user monitoring component 101a and to operate with classifier 307b. Classifier 307b may be a replicate instance of classifier 309a discussed in connection with profile adjustment module 307a, the same classifier 309a resident in profile adjustment module 307a (accessed via e.g. network connection), or a classifier 307b trained substantially similarly to classifier 309a. Route segment analysis module 303b may provide the road features of the route segment in question for a given road user profile to classifier 307b, and obtain an estimated content attention level for each route segment, e.g. $C_1$, $C_2$, and $C_3$ as further depicted by Fig. 4a, the estimated content attention levels being based on respective road link feature types and user attention levels, e.g. as determined by classifier 307b. The road link features of the route segment provided to e.g. the classifier 307b, may be regarded as compatible road link features since these would correspond to the road link feature types (or road link attribute types) used to train the classifier 309a or 307b. In some embodiments, for example if the classifier 307b has been trained to additionally consider contextual information, the classifier 307b may also be provided with contextual information data valid for the time of route traversal (e.g. time of day, weekday, workday/day off, season, other occupants in vehicle, etc.) for obtaining an estimated content attention level.

Fig. 4a depicts an estimated content attention level graph for a user corresponding to a "driver" profile, determined based on road link features of route 111. Route 111, includes a first route segment 111c, in an area with curvy roads and mountainous terrain (as depicted in Fig 1a), on asphalt, in an urban area, with a low-speed limit (30 km/h), with low traffic, under light rain, without autonomous driving availability (for the vehicle in question). The driver may not have full attention available as the road is curvy, the weather is rainy, and the urban area may exhibit heightened activity adjacent to the road. The classifier 307b may then determine, based on user profile and combination of road features, that the content attention level is rated at e.g. $C_1$=0.2 for route segment 111c. Further along the route, for route segment 111d, Autonomous Driving may be available for a relatively straight and fast highway road with low traffic. Under such conditions, the vehicle may take over driving, allowing for the driver-now-passenger to relax and have more available attention. The classifier 307b may determine a corresponding content attention level at e.g. $C_2$=0.7 for route segment 111d. For the last segment of the route, route segment 111e, the road is a gravel road, with a traffic jam (e.g. traffic "high"), with straight road geometry. The vehicle can barely advance due to the traffic jam, such that the driver may be considered attentive, leading the classifier 307b to determine an content attention level of at e.g. $C_3$=0.6. In some embodiments, route segments where the vehicle is not advancing (e.g. gridlock, EV charging, rest stops, mandated legal resting time for professional drivers, etc.) may also correspond to route segments. There may be less distractions for such segments, such that estimated content attention levels are rated as higher (e.g. 0,8-0,9) . Similarly, embodiments may opt for providing the estimated content attention level in terms of equivalent attention level ranges, attention level categories, such as those described in connection with classifier 309a.

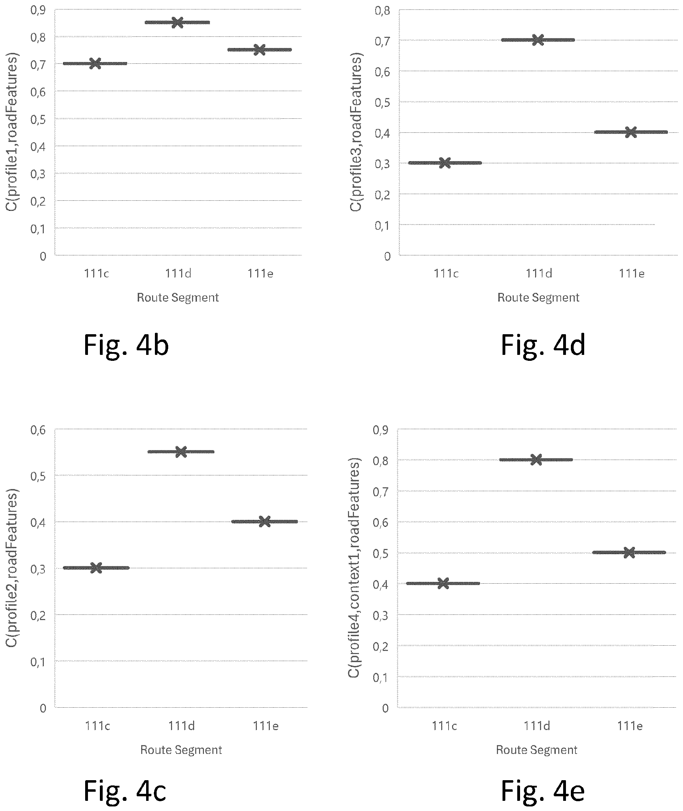

Figs. 4b-4e provide graphs corresponding to examples of classifier 307b outputs. Utilizing different user profiles may lead the classifier 307b to determine different estimated content attention levels for different user types. For example, Fig 4b provides an example estimated content attention level graph for a passenger profile (profile1). Estimated content attention levels may be generally higher than those of a driver as the passenger is not busy with the task of driving. The curvy route segment 111c and the high traffic route segment 111e may cause more distractions than the highway road segment 111d, resulting in variation of the values detected by classifier 307b. Fig. 4c provides an instance of a driver profile (profile2), but without available Autonomous Driving, leading to a lower estimated content attention level for route segment 111d. Fig. 4d provides an instance of a driver profile with autonomous driving (profile3, which may be different to the driver profile utilized in connection with Fig.4a, e.g. "Taylor's" profile), which exhibits a higher estimated content attention level for route segment 111e than profile2. Finally, profile4, which may be a contextual data enhanced version of profiles (or e.g. "Taylor's" profile - contextual), utilized with additional context features (e.g. morning time, weekday, driver alone, in summer) yields different estimated content attention level values for the different route segments. These are only non-limiting examples, and it is envisioned for example embodiments to operate analogously with different combinations of input user profiles, road features, and optionally, with contextual information data.

Content attention profile output module 305b may be configured to receive the estimated user attention levels from classifier 307b and provide these to the GenAI component 103a as a content attention profile. A content attention profile may provide a sequence of estimated content attention level corresponding to a sequence of route segments of a route. In addition, the content attention profile may be expressed as a timing sequence for which an estimated content attention level has been determined. In such cases, the time ranges correspond to estimated traversal times for respective route segments. The content attention output module 305b may query a routing service, such as LBS platform 107, a local routing engine of mobile device 101, etc., and obtain estimated traversal times for the route and for the individual route segments of the route. For example, a content attention profile corresponding to the example route 111 of Fig. 4a may be exemplified by the following data structure:

```
routeContentAttentionProfile={ C1=0.2, t1= 17min;
                                C2=0.7, t2= 18min;
                                C3=0.6, t3= 10min; }
```

where $C_1$ is the estimated content attention level for the first $t_1$ minutes (17min) of the route, $C_2$ is the estimated content attention level for the next $t_2$ minutes (18min), and $C_3$ is the estimated content attention level for the next $t_3$ (10min) minutes. Analogously, the timing sequence may be further expressed as time ranges (e.g. $t_1$=[0m-17m], $t_2$=[17m-35m], $t_3$= [35m-40m]) or any other equivalent representation. Similarly, embodiments may opt for providing the estimated content attention level in terms of equivalent attention level ranges, attention level categories, such as those described in connection with classifier 309a, such that e.g. $C_1$= "concentrated" , $C_2$="attentive" $C_3$="responsive", etc.. Additional information may be provided as part of the content attention profile, such as estimated time of traversal for the complete route, a time buffer (accounting for e.g. for settling in the vehicle, arrival, pauses on route, etc.) and the like.

Attention is now drawn to the GenAI component 103a depicted in Fig. 3c. The GenAI component 103a may be implemented as part of content delivery platform 103, as part of mobile device 101, as part of another service reachable via communication network 109, or may be a stand-alone implementation, also reachable via communication network 109. GenAI component 103a is depicted comprising a media source file reception module 301c, a prompting module 303c, a GenAI core module 305c, and a media target file output module 307c.

The media source file reception module 301c can obtain a source content data file through various means, including the content delivery platform 103 (e.g., a streaming service with a content library), file sharing over communication network 109, mobile device 101 (via USB stick, SD card, or other available storage), direct user input (e.g., voice/video recording, text typing), or any other method capable of providing a content data file. Media content selection, for example initiated by a user over user interface 101b, can occur through browsing a library of available files, uploading a new file, or receiving one from a third-party source. In some embodiments, users can create media content ad-hoc based on recorded input (e.g. typed, voice and/or video recording) via user interface 101b. Content data files can contain various types, such as text files, audio files, or video files. The media files may cover a wide variety of content, such as stories, novels, poetry, textbooks, scientific papers, technical papers, marketing prospects, speeches, audiobooks, podcasts /talk radio broadcasts, music (e.g. playlists, albums, symphonic works, operas, etc.), video clips, music videos, TV shows, tutorials, soap operas, feature films, etc. Media source file reception module 301c may also (re-)format the incoming source content data file into a format compatible with the GenAI core module 305. For example, source formats may comprise MP4, AVI, MOV, MKV, MP3, WAV, FLAC, OGG, PDF, DOCX, TXT, EPUB, MOBI, AZW, and the like, but GenAI core module 305 may only be compatible with e.g. TXT for text, WAV for audio, or AVI for video. File format converters or libraries, well-known in the art, may be utilized to (re-)format the incoming source content file. In addition, media source file reception module 301c may further provide metadata pertaining to the content, in particular metadata providing information about media content length. For example, a word count or an estimated time to read (based in e.g. a words per minute metric) may be provided for a text file, a playback duration for an audio or video file. In another example, content section markers such as sections, chapters, scenes, acts, etc. may also be provided. The media source file reception module 301c may be further configured to provide the source content data file to the GenAI core module 305c.

GenAI component 103 is further depicted comprising prompting module 303c. Prompting module 303c may be configured to receive a content attention profile, such as one provided by content attention profile output module 305b. The prompting module 303c may also comprise or have access to a prompt library. The prompt library may comprise a wide variety of prompt instructions, which may be provided to the GenAI core module 305c to adjust the level of detail of a content file. The following non-limiting examples for text modification prompts may be stored in the prompt library:

1. Simplify:

   1.1. Cut out:

      1.1.1. Repeated ideas or concepts

      1.1.2. Transitions between sentences (e.g., "however," "in addition")

   1.2. Use active voice only: Convert passive voice constructions to active voice, as they often contain unnecessary words.

   1.3. Remove redundant information: Omit details that are already implied by other text or that don't add new meaning.

   1.4. Reduce sentence length: Shorten sentences to improve clarity and flow.

   1.5. Eliminate jargon and technical terms: Replace specialized language with more general, everyday expressions.

   1.6. Simplify complex concepts: Break down abstract ideas into simpler, more concrete explanations.

   1.7. Use simpler vocabulary:

      1.7.1. Replace words with one or two syllables

      1.7.2. Avoid rare or obscure words

   1.8. Merge similar sentences: Combine multiple related sentences into a single sentence for better cohesion.

   1.9. Omit filler words and phrases: Remove unnecessary words like "really," "actually," or "basically."

   1.10. Use concise paragraphs: Break up long paragraphs into shorter ones, each with a clear purpose.

2. Expand:

   2.1. Add concrete examples: Include real-world illustrations to help readers better understand complex ideas.

   2.2. Provide more context: Give background information or historical context to make the text more informative and engaging.

   2.3. Break down complex concepts: Divide abstract ideas into smaller, more manageable parts for easier comprehension.

   2.4. Include counterarguments: Address opposing views to demonstrate depth of thought and encourage critical thinking.

   2.5. Add relevant anecdotes: Share personal stories or examples to illustrate key points and make the text more relatable.

   2.6. Define technical terms: Explain specialized language to ensure readers understand the content.

   2.7. Use transitional phrases: Connect ideas between sentences and paragraphs with words like "meanwhile," "in contrast," or "nevertheless."

   2.8. Include subheadings or headings: Organize the text into logical sections for easier reading and comprehension.

   2.9. Add supporting evidence: Cite credible sources, data, or research to back up claims and strengthen arguments.

2.10. Explore related topics: Discuss tangential ideas that expand on the main topic and provide additional insights.

3. Condense:

3.1. Combine similar information: Merge duplicate concepts into a single idea or summary statement.

3.2. Eliminate unnecessary words: Remove adverbs, adjectives, or phrases that don't add meaning to the text.

3.3. Simplify sentence structures: Use shorter sentences with fewer clauses for easier reading.

3.4. Use concise language: Replace complex vocabulary with simpler alternatives.

3.5. Omit repetitive information: Skip redundant details and focus on essential points.

3.6. Cut out unnecessary examples: Remove anecdotal evidence that doesn't add to the main message.

3.7. Streamline lists and bullet points: Condense lengthy lists into shorter, more digestible versions.

3.8. Remove transitional words: Use fewer transition words like "however," "therefore," or "consequently" to improve flow.

3.9. Use active voice consistently: Avoid passive voice constructions that can make the text appear less concise.

3.10. Focus on key takeaways: Emphasize main points and leave out supporting details for a more condensed version.

4. Enrich

4.1. Use metaphors or analogies: Compare complex ideas to relatable concepts or experiences to facilitate understanding.

4.2. Add historical or cultural context: Incorporate relevant background information to provide deeper insight into the topic.

4.3. Explore nuanced perspectives: Discuss multiple viewpoints and controversies related to the main idea.

4.4. Incorporate data or statistics: Use credible sources to support arguments with concrete evidence.

4.5. Include expert opinions: Quote experts or thought leaders in the field to add credibility and depth.

4.6. Use sensory language: Incorporate descriptive language that appeals to multiple senses (sight, sound, touch, etc.) to engage readers more fully.

4.7. Explore philosophical or existential implications: Discuss deeper questions or underlying assumptions related to the main idea.

4.8. Add literary devices: Use poetic language, allusions, or rhetorical devices to enrich the text and create a more engaging reading experience.

4.9. Incorporate case studies or examples: Use real-world illustrations to demonstrate key concepts in action.

4.10. Encourage critical thinking: Include questions, puzzles, or brain teasers that challenge readers to think more deeply about the topic.

5. Streamline

5.1. Use clear headings and subheadings: Organize the text into logical sections with concise titles.

5.2. Simplify formatting: Use standard fonts, margins, and line spacing for easier reading.

5.3. Eliminate unnecessary graphics or images: Remove visual elements that distract from the main message.

5.4. Cut out unnecessary white space: Reduce blank lines or paragraphs to improve flow and efficiency.

5.5. Use bullet points instead of lists: Condense lengthy lists into concise bullet points for better readability.

5.6. Streamline navigation and links: Organize the text with logical headings and clear calls-to-action.

5.7. Use concise titles and summaries: Write attention-grabbing headlines and brief summaries to entice readers.

5.8. Eliminate unnecessary appendices: Remove supplementary materials that aren't essential to understanding the main message.

The aforementioned prompt examples are only provided for illustration and are not intended to limit the multiple different possible prompts that may be contained in the prompt library. The prompt examples are in particular suited for application on text files. However, analogous prompts may be utilized for operating on narrated texts as audio files. In such embodiments operating on audio files, other prompts may be utilized in addition, such as:

6. Narrated text editing:

6.1. Cut out unnecessary pauses: Remove excessive silence or breathing sounds that disrupt the flow.

6.2. Simplify sound effects: Reduce the number and intensity of sound effects, such as echo, reverb, or background noise.

6.3. Streamline music or jingles: Cut down on repetitive or unnecessary musical elements to focus on the main message.

6.4. Use more concise voiceovers: Limit the length of spoken content to keep listeners engaged and focused.

6.5. Remove filler words and phrases: Eliminate unnecessary words like "um," "ah," or "you know" that can distract from the message.

6.6. Cut out tangential conversations: Remove conversations that don't contribute to the main topic or narrative.

6.7. Simplify soundscapes: Reduce background noise or sound effects that aren't essential to the story or message.

6.8. Use more direct dialogue: Cut down on unnecessary dialogue, such as filler words or repetitive phrases, to make it more concise and engaging.

6.9. Remove unnecessary sound transitions: Eliminate abrupt or jarring sound effects when transitioning between segments or ideas.

6.10. Enhance clarity with compression: Use audio compression techniques to even out volume levels and improve overall clarity.

When applying example embodiments on a content data file containing music, prompts such as the following may be provided in the prompt library:

7. Music editing

7.1. Simplify melody: Reduce repetition or redundancy in the melody, making it more concise and engaging.

7.2. Cut out unnecessary harmonies: Eliminate excessive or repetitive harmonic textures to improve clarity and focus on the main melody.

7.3. Streamline instrumentation: Remove or reduce unnecessary instrumental parts to enhance the overall sound and impact of the piece.

7.4. Enhance contrast with dynamics: Use dynamic range to create more contrast and interest in the music, making it more engaging and emotive.

7.5. Refine tempo and rhythm: Synchronize or adjust the tempo and rhythm of individual tracks to improve overall cohesion and flow.

7.6. Simplify chord progressions: Reduce complexity or repetition in chord progressions, making them more effective and impactful in conveying emotions or moods.

Similarly, for video files, prompts such as:

8. Video editing

8.1. Remove unnecessary footage: Cut out sections of footage that aren't essential to the story or message, such as redundant shots or filler scenes.
8.2. Simplify transitions: Reduce the number and type of transitions between clips, using smoother and more cohesive connections instead.
8.3. Streamline dialogue: Remove unnecessary dialogue, such as filler words or repetitive phrases, to make it more concise and engaging.
8.4. Enhance pacing with timing adjustments: Adjust the timing of individual clips to improve overall flow and pacing, making the video more engaging and dynamic.
8.5. Refine scene transitions: Synchronize or adjust the fade-ins/fade-outs between scenes, removing awkward or abrupt transitions instead.
8.6. Simplify color correction: Reduce excessive brightness, contrast, or saturation, allowing for a more natural and cohesive visual tone throughout the clip.

may be available in the prompt library for operating on a video file. The prompt library may further include definitions for which prompts are suitable for a particular media type.

The prompting module 303c may receive source file duration information (e.g. in the form of metadata) from the media source file reception module 301c and the content attention profile (e.g. from route analysis component 107a), containing estimated content attention level for respective estimated traversal times of route segments. Initially, a source file content duration $Cd_{total}$, may be split in content time durations $Cd_1$, $Cd_2$, ... $Cd_N$, corresponding to estimated traversal times for respective route segments $RS_1$, $RS_2$,... $RS_N$. Alternatively, if content section markers such as sections, chapters, scenes, acts, etc. are present in the content, the content section markers and their corresponding timings may be used as content time durations $Cd_1$, $Cd_2$, ... $Cd_N$. In such cases, it may be beneficial to assign more than one chapter marker to a route segment (or vice versa) if e.g. a route traversal time for one route segment is sufficient to accommodate more than one content section.

A rule set of the prompting module 303c may provide for application of different prompts from the prompt library to be used for different values of estimated content attention level , route segment estimated traversal times and source file duration information. For example, the rule set may provide for building a control prompt for a content time duration $Cd_1$, which is shorter than the estimated traversal time for route segment $RS_1$, i.e. $Cd_1 < RS_1$, and for which an estimated content attention level corresponds to a range of e.g. [0,7-0,9] (or "highly attentive", in e.g. attention category terms), to utilize a prompt example like "*2.6 Expand - Define technical terms*". The prompting module may then build a control prompt such as "for *the first [CD₁] minutes of the content, expand the content* to *[RS₁] minutes of duration. To do so, **define technical terms**"*. In another example, for example for a content time duration $Cd_1$, which is longer than the estimated traversal time for route segment $RS_1$, i.e. $Cd_1 > RS_1$, and for which an estimated content attention level corresponds to a range of e.g. [0,7-0,9] (or "highly attentive", in e.g. attention category terms), to utilize a prompt example like 3.10 Condense - Focus on key takeaways. The prompting module may then build a control prompt such as *"for the first [CD₁] minutes of the content,* **condense** *the content to [RS₁] minutes of duration. To do so,* **focus on key takeaways"**. Similar rules may be defined for a myriad of combinations for different estimated content attention level ranges or categories, duration of content sections or route segments. Similarly, bespoke rules may be further included to manage specific media types, such as music, audiobooks, video clips, etc. utilizing prompt examples such as prompt examples 6.- 8. (narrated text, music, video). To include variation, some choices of example prompts may be randomized. For example, a main prompt category (e.g. "streamline") may be defined in the rule, but a randomized choice of example within the category may be utilized. Other randomizations are possible, for which the prompting module may further define rules that veto certain combinations, e.g. $Cd_1 < RS_1$ not combinable with "streamline",or "prompts of category [8. Video editing] not combinable with source media type (text)". The provided examples are for illustration of the multiple possibilities for implementation of example embodiments and are not intended to limit the extent or content attention of the rule set utilized by the prompting module 303c.

In some embodiments, prompting module 303c may also be configured to receive user preference information for generating additional control prompts, for example control prompts pursuant to (further) adjusting the content's level of detail. User preferences may be received as responses to pre-formulated questions, for example via interaction with user interface 101b. For example, preferences such as content priority (e.g. - "Do you prefer to listen to the entire podcast or just the key highlights?" - "Which sections of the podcast are most important to you (e.g., interviews, expert opinions, story sections)?"), narration speed (e.g. "At what speed do you find spoken audio still comfortable to understand?", for example measured as 1x, 1.2x, 1.5x playback speed, 100-120 words per minute/wpm, 121-160 wpm, 161-200 wpm, "slow", "regular", "fast", etc.), topics of interest (e.g. - "Are there specific topics or segments within this podcast that you are (or not) particularly interested in?", "Would you like to skip any recurring segments or advertisements?"), content editing tradeoffs (e.g. "Would you sacrifice some detail for a shorter listening time?", "What's more critical for you: maintaining original content integrity or fitting the podcast into your available listening time?") and the like. Control prompts may be generated by prompting module 303c such as, "focus on highlights"," speak at 121-160 wpm", "do not provide expert opinions", "please include as much content as possible to fit the <totalRouteDuration>" and the like. Furthermore, prompting module 303c may learn previously provided user preference information, associate it with a user profile, and apply similar prompts during new route instances. Prompting module 303c may also further adjust user preferences in response to feedback user interactions, such as - *"How did you find the speed adjustment of your last podcast? Would you like it faster, slower, or was it just right?"* or - *"Was the summarized content sufficient, or did you feel you missed out on important details?"*.

Prompting module 303c may further provide additional control prompts, such as "Modify the media file content such that it has a duration of <totalRouteDuration>", where totalRouteDuration duration corresponds to the estimated route duration for the complete route (as e.g. calculated by a routing engine, LBS platform 107, or the like). This allows for a so-called "magical moment", where a complete content piece may be enjoyed in sync with the duration of the total route traversal time, creating a feeling that no time was wasted by simply getting from A to B. totalRouteDuration may be further modified to consider a buffer time (e.g. for boarding/settling in vehicle, arriving, pauses, etc.), such that the content can be enjoyed in its entirety without intervening in other activities. While pauses may also be embedded in the content in applying example embodiments, known playback pause controls may also be utilized. The prompting module 303c may be further configured to provide at least one prompt to the GenAI core module 305c. In addition, the prompting module 303c may also generate prompts that allow for change from one media type to another, e.g. "turn this scientific paper into a podcast.", or "create a music video for this album". Such prompts pursuant to media type changes may be generated in response to a user input (e.g. via user interface 101b) or based on a stored user preference. In some embodiments, the prompting module 303c may further include rule sets corresponding to the user profiles discussed in connection with the user profile module 303a.

The GenAI core module 305c, depicted as part of the GenAI component 103a, may be configured to receive a source content data file (e.g. from media source file reception module 301c) and further receive control prompts (e.g. from prompting module 303c). The GenAI core module 305c, may not necessarily be co-located within the GenAI component 103a itself, or as part of the content delivery platform 103, it may be implemented as a stand-alone solution on independent cloud infrastructure accessible over e.g. communication network 109. Examples of GenAI solutions akin to GenAI core module 305c may be offline GenAI services such as Ollama (Open Source from Meta), GenAI services such as ChatGPT (OpenAI), Gemini from Google, DALL-E (OpenAI), Synthesia, Canva AI, VeedIO and the like, implementing AI models such as Llama, Mistral, GPT, DALL-E, BARD, Veo, Imagen, etc. In general, GenAI core module 305c may obtain a piece of content (e.g. from an input data file) and generate, based on control prompts (provided e.g. via user interface, API, etc.), an edited output data file which is based on the content of the input data file. Different GenAI core module implementations may provide for multi-modal inputs, where input and/or output formats may be text, sound or video formats, such that the GenAI core module 305 can effectively accept an incoming source content file, process it in line with the constrains specified in the control prompts, and provide an output content file. In some embodiments the target output format may be of the same type (e.g. video-> video) or of a different type than the source content file (e.g. text-> audio).

Media target file output module 307c is further depicted as part of the GenAI component 103a. Media target file output module 307c is configured to receive the edited output data file from the GenAI core module 305c, if necessary, (re-) format it in a compatible file type usable by the mobile device 101 for presentation via the user interface 101b, and provide it e.g. via communication network 109 to the mobile device 101 as an output content data file. In some embodiments, the output content data file may also be provided for storage for later presentation, optionally with a reference to the route for which it was generated. For example, a piece of content may have caused a particular impression on a user, in part by being presented during a route that matched well with the content. The user may want to re-live the experience and enjoy the content on the same route again, such that having the content stored with a reference to the route enables easy selection/playback of the content. Also, a processing-intensive tasks like content generation can be done in advance prior to route traversal, such that an output content data file may be stored for future route traversal.

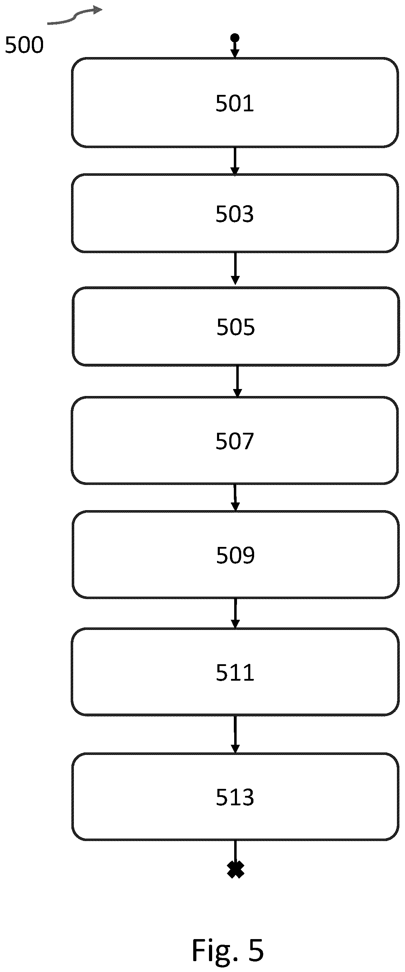

Moving on to Fig. 5, a flowchart depicting a method 600 of example embodiments is provided.

In step 501, a route for a user based on an origin location and a destination location, the route comprising a plurality of road links, is obtained. In Example embodiments, a route such as route 111 may be obtained by route analysis component 107a.

In step 503 a content attention profile associated with the route, via a route analysis component (e.g. route analysis component 107a), is generated. The content attention profile is generated by assigning an estimated content attention level to each of the road links of the plurality of road links based on a set of based on a correlation of one or more road link feature types of the respective road link with a user attention level corresponding to the one or more of road link feature types. In example embodiments, classifier 307b may be used to assign the estimated content attention level to road links of route 111.

In step 505 a first content data file is obtained, wherein the first content data file comprises one or more of text, audio and video data. In example embodiments, the content delivery platform 103 may obtain the first content data file by e.g. retrieval from a content library, via user input, file sharing, etc.

In step 507 a control prompt based on the first content data file and the content attention profile is generated, wherein the control prompt comprises instructions for adjusting a level of detail of at least a portion of the content of the first content data file is generated. In example embodiments, the control prompt may be generated by the prompting module 303c.

In step 509 the first content data file and the control prompt are provided to a GenAI component, e.g. GenAI component 103a. In example embodiments the source content data file and the control prompt may be provided respectively by the media source file reception module 301c and the prompting module 303c to the GenAI core module 305c.

In step 511 a second content data file is obtained from the GenAI component. The second content data file is generated based on the first content data file. In example embodiments, the output content file may be obtained from the media target file output module 307c by the mobile device 101.

In step 513 presentation of the second content data file on a device associated with the user during traversal of the route is caused. In example embodiments, the output content file may be presented via the user interface 101b.

Some example embodiments may perform the aforementioned method only prior to route traversal. However, it is envisioned that other example embodiments may also continue to update the output content file while the route is being traversed. For example, changing route features (e.g. a traffic level, pedestrian density level, noise level, variable speed limit, construction zone, autonomous driving availability indication, accident hazard indication, etc.) provided by e.g. LBS platform 107, may affect the resulting content attention profile, such that estimated content attention levels may be different to those calculated prior to departure. In another example, detours, route recalculation, engaging/disengaging Autonomous Driving (or other ADAS functions), etc., may also lead to changes in the content attention profile. Similarly, a user attention level (obtained from e.g. the user monitoring module 101a) while traversing a first route segment may be utilized to adjust the estimated content attention levels for the next route segment(s). In such cases, the GenAI component 103a may be provided with a revised content attention profile, and an instruction to provide a new output content file corresponding to the remaining portion of the route.

In yet some example embodiments, information about a critical safety condition (e.g. accident event, near-miss, failing vehicle system, or the like) may be obtained from the vehicle 120, the mobile device 101, the LBS platform, etc. It is expected that under critical safety conditions, user attention may be otherwise occupied with e.g. dealing with the stress of the situation. In such cases, example embodiments may provide for pausing content playback at the time of the critical condition and provide instructions to the GenAI component 103a to generate content re-iterating portions that were mentioned within a buffer time distance of the critical condition, such that the user may resume content consumption without losing track. Optionally, the GenAI component 103a may further provide for generation of a recap of the previously presented content, before resuming content playback at the point of the critical condition.

In some embodiments, a content comprehension evaluation may be administered to the user of mobile device 101 after presentation of the output content file. The content comprehension evaluation may be provided as a quiz, game or similar form of interaction with the user, during which the user provides input, for example via user interface 101b. The content comprehension evaluation may help in determining which sections of the content were, for example, well understood, oversimplified, not sufficiently described or not correctly understood by the user. The user replies may be then useful to obtain a revised estimated content attention level of the user for the route segments after traversing the route, or in some cases still during route traversal (e.g. when the quiz is provided after the content but still on route). By determining the point of time during presentation where the information of the content to be evaluated was presented, some embodiments may be enabled to obtain the corresponding road link features of the route segment traversed at the time, from e.g. route analysis component 107a.

Providing the revised estimated content attention level(s) together with the corresponding road link features may enable, in some embodiments, an adjustment of the correlation of the at least one or more road link feature types with the user attention level based on the obtained user input. The data may be, for example, provided to the classifier 309a or 307b for "fine-tuning" of the supervised machine learning model in connection with a user profile corresponding to the user that provided input for the comprehension evaluation. Similarly, the revised estimated content attention levels may be utilized to update or modify a rule set of the prompting module 303c.

In yet some other embodiments, a content attention profile may be further generated based on a collective user attention level for a plurality of users of the device. For example, if it is detected that multiple users are on board a vehicle (e.g. by user monitoring module 101a), user profiles corresponding to respective users may be found in e.g. user profile module 303a. The user profile module 303a may be further configured to determine a collective user attention level by averaging attention levels, weighting attention levels (e.g. prioritizing passengers over drives, children over adults, etc.) or determining that the collective user attention level is equal to the user profile with the lowest (or highest) attention level value.

It should be understood that, while embodiments have been described in connection with a vehicle 120 resembling a personal vehicle (e.g. car, minivan, SUV, motorcycle, etc.), embodiments may similarly be applied to other means of transport, such as bicycles, trucks, trains, boats, airplanes, or the like. In some of such cases availability of sensor data observations may be limited to sensor data obtainable by the mobile device of the user e.g. a smartphone with only camera, microphone or vital signs tracking (via wearable device). In other such embodiments, route information may be limited to phases/milestones of the route, e.g. in aerial travel on/offboarding, taxiing, take-off, cruise, approach, landing, etc.; in water on/offboarding, harbor navigation, shipping lanes, open water, etc.; in terrestrial public transport on/offboarding of the user, on/offboarding of other public transport users, occupancy levels, noise levels, switching means of transport (e.g. bus A-> bus B), etc.. Still, associated attention levels may be determined based on current or previously collected sensor observations while traversing a route segment, in connection with an associated user profile (e.g. "train-passenger", "bicycle-rider", etc. ) which may be similarly utilized to control content generation based on route information.

Another suitable area of application for example embodiments may be pedestrian use, where no vehicle is involved. A user may still traverse a route for which route segment features are derivable from e.g. adjacent road link features, walking path link features, etc., and sensor observations are available from e.g. a smartphone or a wearable device. A user profile corresponding to a pedestrian may be utilized analogously to aforementioned profiles like, driver, passenger, etc. Estimating content attention levels for such pedestrian routes and generating a corresponding content attention profile may similarly provide for controlled content generation based on route information.

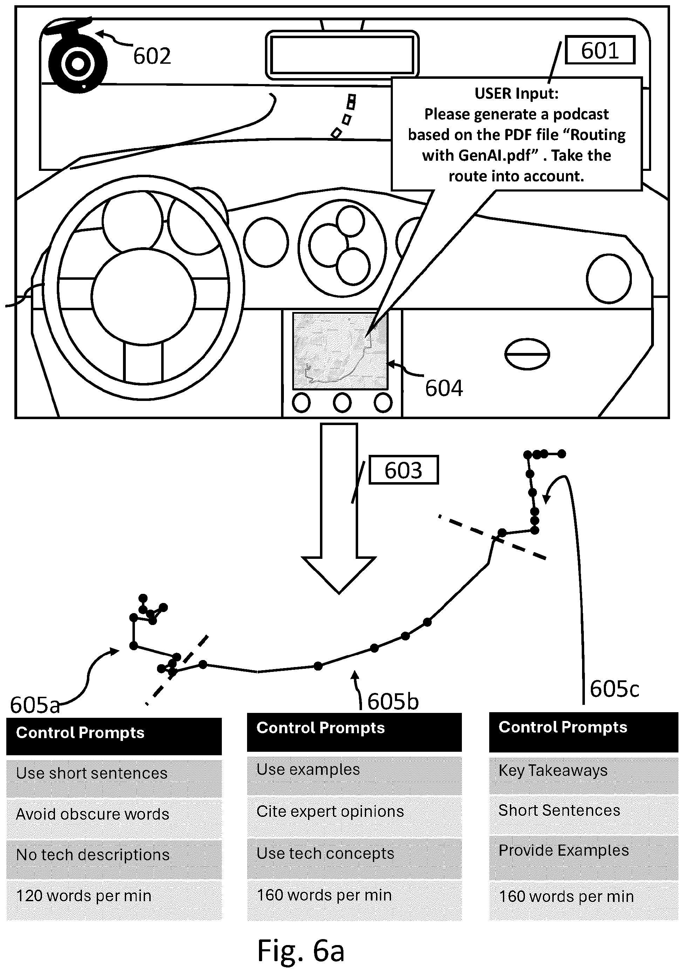

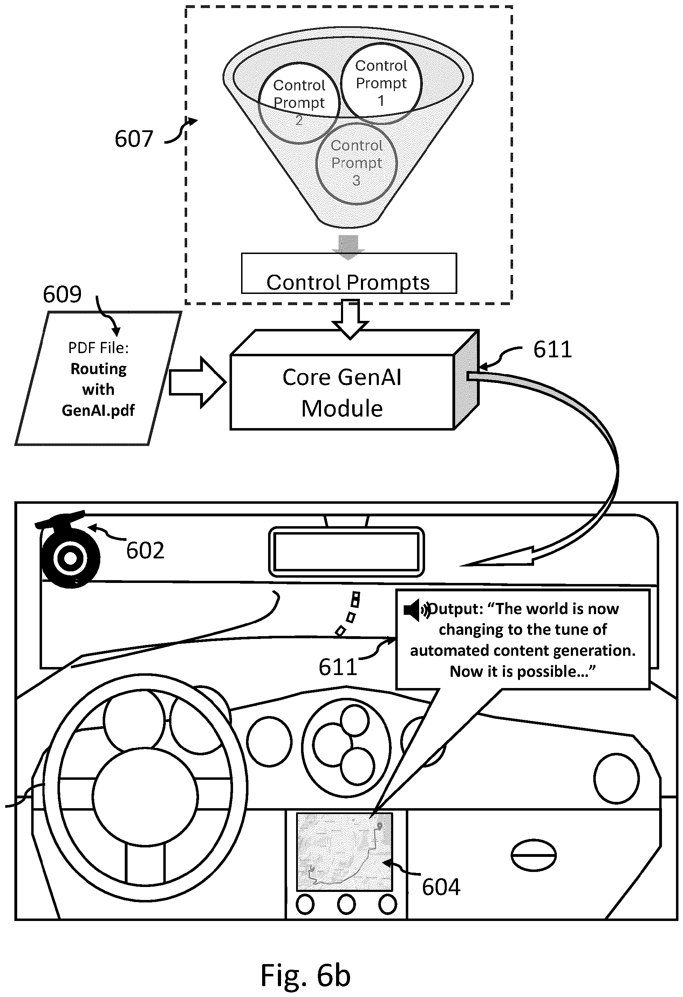

Figs. 6a and 6b describe an operational example in accordance with example embodiments. The example is provided as an illustration and should not be considered as limited. In addition, some interactions with aforementioned components are omitted to increase readability. A user onboard a vehicle, such as vehicle 120, may request that the vehicle provides a podcast media file based on a PDF file called "Routing with GenAI.pdf". The user may indicate that the podcast may be generated taking into account the route to be traveled, as depicted by dialog box 601, for example by interaction via screen 604 (part of a user interface 101b). In some cases, the instruction may be provided implicitly, e.g. by mere selection of a file to process.

Screen 604 depicts a route to be traveled, for which the podcast should be generated. The route may be made available for analysis, by e.g. a route analysis component 107a, as illustrated by arrow 603. By applying example embodiments, control prompts corresponding to different route sections 605a-c may be generated. In section 605a, the curvy, urban road sections result in a set of content simplification prompts, in section 605b the availability of autonomous driving allows for enrichment of the content, utilizing a higher speech speed, while section 605c may provide for a short but exemplified discussion of the content corresponding to the final portion of the route.

The control prompts may be then provided (e.g. at 607) to a core GenAI module (such as GenAI core module 305c) along with the PDF file 609. The core GenAI module may then pass an output podcast file to the user interface of the vehicle, so that it can be narrated e.g. via speakers of the vehicle, as depicted by dialog box 611.

While on route or before starting, user observation sensors, such as camera 602 may monitor the user(s) of the vehicle to determine a current attention level. In such cases, the GenAI component 103a may be provided with a revised content attention profile, and an instruction to provide a new output content file corresponding to the remaining portion of the route. By applying previously described embodiments, the podcast may be adjusted based on the estimated content attention levels to at least increase the chances of proper content comprehension.

In other examples, content may be selected from e.g. streaming services (e.g. YouTube, Spotify, Netflix, etc.) and adjusted for output during the route to be traversed. As GenAI is also capable of generating stories with a few prompted indications, (e.g. *"tell me a 20-minute story about a bunny jumping through a meadow")* short prompts from a user may be provided in lieu of a text file, for example using speech-to-text capabilities.

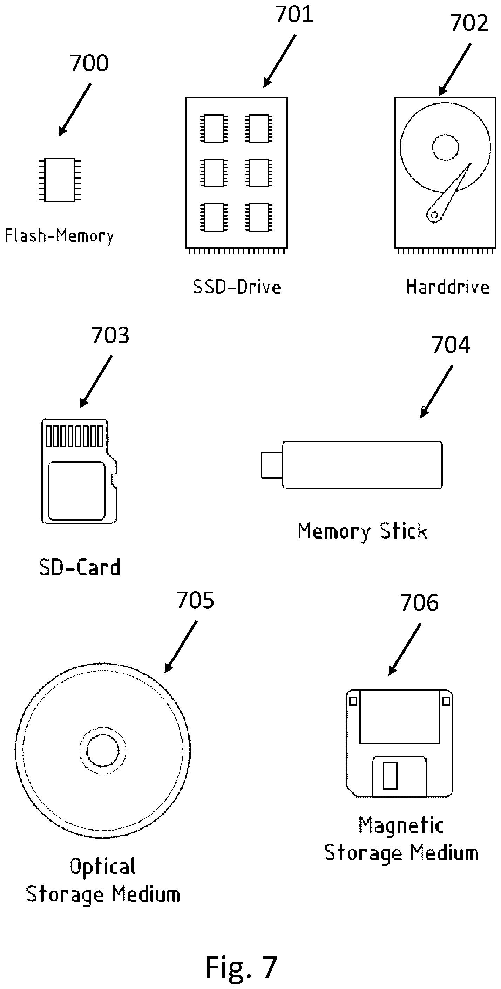

Fig. 7 is a schematic illustration of examples of tangible and non-transitory computer-readable storage media according to the present invention that may for instance be used to implement memory device 203 of apparatus 200 Fig. 2, or any apparatus in accordance with the exemplary aspects disclosed herein. Fig. 7 shows a flash memory 700, which may for instance be soldered or bonded to a printed circuit board, a solid-state drive 701 comprising a plurality of memory chips (e.g. Flash memory chips), a magnetic hard drive 702, a Secure Digital (SD) card 703, a Universal Serial Bus (USB) memory stick 704, an optical storage medium 705 (such as for instance a CD-ROM or DVD) and a magnetic storage medium 706.

In the present specification, any presented connection in the described embodiments is to be understood in a way that the involved components are operationally coupled. Thus, the connections can be direct or indirect with any number or combination of intervening elements, and there may be merely a functional relationship between the components.

Moreover, any of the methods, processes and actions described or illustrated herein may be implemented using executable instructions in a general-purpose or special-purpose processor and stored on a computer-readable storage medium (e.g., disk, memory, or the like) to be executed by such a processor. References to a 'computer-readable storage medium' should be understood to encompass specialized circuits such as FPGAs, ASICs, signal processing devices, and other devices.

The expression "A and/or B" is considered to comprise any one of the following three scenarios: (i) A, (ii) B, (iii) A and B. Having the same meaning as the expression "A and/or B", the expression "at least one of A or B" may be used herein. Furthermore, the article "a" is not to be understood as "one", i.e. use of the expression "an element" does not preclude that also further elements are present. The term "comprising" is to be understood in an open sense, i.e. in a way that an object that "comprises an element A" may also comprise further elements in addition to element A.

It will be understood that all presented embodiments are only exemplary, and that any feature presented for a particular example embodiment may be used with any aspect on its own or in combination with any feature presented for the same or another particular example embodiment and/or in combination with any other feature not mentioned. In particular, the example embodiments presented in this specification shall also be understood to be disclosed in all possible combinations with each other, as far as it is technically reasonable and the example embodiments are not alternatives with respect to each other. It will further be understood that any feature presented for an example embodiment in a particular category (method/apparatus/computer program/system) may also be used in a corresponding manner in an example embodiment of any other category. It should also be understood that presence of a feature in the presented example embodiments shall not necessarily mean that this feature forms an essential feature and cannot be omitted or substituted.

The statement of a feature comprises at least one of the subsequently enumerated features is not mandatory in the way that the feature comprises all subsequently enumerated features, or at least one feature of the plurality of the subsequently enumerated features. Also, a selection of the enumerated features in any combination or a selection of only one of the enumerated features is possible. The specific combination of all subsequently enumerated features may as well be considered. Also, a plurality of only one of the enumerated features may be possible.

The sequence of all method steps presented above is not mandatory, also alternative sequences may be possible. Nevertheless, the specific sequence of method steps exemplarily shown in the figures shall be considered as one possible sequence of method steps for the respective embodiment described by the respective figure.

The subject-matter has been described above by means of example embodiments. It should be noted that there are alternative ways and variations which are obvious to a skilled person in the art and can be implemented without deviating from the scope of the appended claims.

**Claims**

1.  A computer-implemented method, the method comprising:

    obtaining (501) a route for a user based on an origin location and a destination location, the route comprising a plurality of road links;
    generating (503) a content attention profile associated with the route, via a route analysis component, by assigning an estimated content attention level (C1, C2, C3) to each of the road links of the plurality of road links based on a correlation of one or more road link feature types of the respective road link with a user attention level corresponding to the one or more of road link feature types;
    obtaining (505) a first content data file, wherein the first content data file comprises one or more of text, audio and video data;
    generating (507) a control prompt based on the first content data file and the content attention profile, wherein the control prompt comprises instructions for adjusting a level of detail of at least a portion of the content of the first content data file;
    providing (509), to a generative artificial intelligence, GenAI, component (103a), the first content data file and the control prompt;

obtaining (511), from the GenAI component (103a), a second content data file, wherein the second content data file is generated based on the first content data file and the control prompt; and

causing (513) presentation of the second content data file on a device associated with the user during traversal of the route.

2. The method of claim 1, wherein the user attention level is determined based on:

a) an alertness level of a human subject, the alertness level being determined via sensor observations made onboard a vehicle while the vehicle is traversing a road link having road link features corresponding to the one or more road link feature types, and

b) a user profile.

3. The method of claim 2, wherein the user profile is one of a driver profile, a passenger profile, a manually controlled vehicle driver profile, a semi-autonomous vehicle driver/passenger profile, or an autonomous vehicle passenger profile.

4. The method of any preceding claims 2 or 3, wherein the sensor observations comprise gaze tracking, heart rate, breathing rate, speech monitoring, vehicle control input, or a combination thereof.

5. The method of any preceding claim 2 to 4, wherein the sensor observations are based on the user and/or one or more human subjects other than the user.

6. The method of any preceding claim, wherein the correlation between the one or more road link feature types and the user attention level is learned using a supervised machine learning technique.

7. The method of any preceding claim, wherein the content attention profile is further generated based on a collective user attention level for a plurality of users of the device.

8. The method of any preceding claim, wherein the at least one or more road link feature types comprise at least one of a road geometry, a lane count, a functional classification, a speed limit, a road surface type, a road surface hazard indication, an autonomous driving suitability indication, or a geographical area designation.

9. The method of any preceding claim, wherein the at least one or more road link feature types further comprise at least one of a traffic level, a weather condition, a pedestrian density level, a noise level, a variable speed limit, a construction zone, an autonomous driving availability indication, or an accident hazard indication.

10. The method of any preceding claim, wherein the origin location is a current device location, a predicted origin location, or a user input origin location, and wherein the destination location is a predicted destination location, or a user input destination location.

11. The method of any preceding claim, further comprising:

associating the route with the second content data file; and,

storing the second content data file for presentation during a future route traversal.

12. The method of any preceding claim, further comprising:
providing, to the GenAI component (103a), an estimated route traversal time to adjust the duration of the second content data file.

13. The method of any preceding claim, wherein the control prompt further comprises instructions for providing the second content data file of a media type different to the first content data file.

14. The method of any preceding claim, further comprising:

requesting user input based on the content of the second content data file;

obtaining the user input from the user; and,

adjusting the correlation of the at least one or more road link feature types with the user attention level based on the obtained user input.

15. A computer program product comprising instructions which, when executed, perform a method according to claims 1-14.

16. An apparatus (200) comprising means for executing a method according to claims 1-14.

**Fig. 1**

**Fig. 1a**

Fig. 1b

Fig. 2

101a

301a

303a

305a

307a

309a

Fig. 3a

107a

301b

303b

305b

307b

Fig. 3b

103a

301c

303c

305c

307c

Fig. 3c

| 111c | |
|---|---|
| roadGeom | "Highly curved" |
| area | "Urban" |
| traffic | "Low" |
| speedLimit | "30 km/h" |
| roadSurface | "Asphalt" |
| autonDriving | No |
| Weather | "Light rain" |
| ... | ... |

-> $C_1 = .2$

| 111d | |
|---|---|
| roadGeom | "straight" |
| area | "rural" |
| traffic | "Low" |
| speedLimit | "120 km/h" |
| roadSurface | "Asphalt" |
| autonDriving | yes |
| Weather | "dry" |
| ... | ... |

-> $C_2 = .7$

| 111e | |
|---|---|
| roadGeom | "straight" |
| area | industrial |
| traffic | "high" |
| speedLimit | "50 km/h" |
| roadSurface | "gravel" |
| autonDriving | No |
| Weather | "dry" |
| ... | ... |

-> $C_3 = .5$

Fig. 4a

Fig. 4b

Fig. 4d

Fig. 4c

Fig. 4e

Fig. 5

602

601

USER Input:
Please generate a podcast
based on the PDF file "Routing
with GenAl.pdf" . Take the
route into account.

604

603

605a

605b

605c

| Control Prompts |
| --- |
| Use short sentences |
| Avoid obscure words |
| No tech descriptions |
| 120 words per min |

| Control Prompts |
| --- |
| Use examples |
| Cite expert opinions |
| Use tech concepts |
| 160 words per min |

| Control Prompts |
| --- |
| Key Takeaways |
| Short Sentences |
| Provide Examples |
| 160 words per min |

Fig. 6a

Fig. 6b

Fig. 7

Europäisches
Patentamt

European
Patent Office

Office européen
des brevets

## EUROPEAN SEARCH REPORT

**Application Number**

EP 25 15 0221

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2019/212160 A1 (KENNEDY LAWRENCE [US] ET AL) 11 July 2019 (2019-07-11) | 1-10, 12-16 | INV. G01C21/36 |
| A | * paragraph [0107] - paragraph [0127]; figures 9,12,13 * | 11 | |
| Y | WO 2024/159241 A2 (ELEVENTHIRTEEN LLC [US]) 2 August 2024 (2024-08-02) * paragraph [0144] - paragraph [0146] * | 1-10, 12-16 | |
| Y | US 2023/150551 A1 (BEAUREPAIRE JEROME [DE] ET AL) 18 May 2023 (2023-05-18) * paragraph [0047]; claims 1-3 * | 4,5 | |

**TECHNICAL FIELDS SEARCHED (IPC)**

G01C

The present search report has been drawn up for all claims

| Place of search | Date of completion of the search | Examiner |
|---|---|---|
| The Hague | 17 June 2025 | Chapple, Ian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 0221

This annex lists the patent family members relating to the patent documents cited in the above-mentioned European search report.
The members are as contained in the European Patent Office EDP file on
The European Patent Office is in no way liable for these particulars which are merely given for the purpose of information.

17-06-2025

| Patent document cited in search report | Publication date | Patent family member(s) | Publication date |
|---|---|---|---|
| US 2019212160 A1 | 11-07-2019 | US 2018188054 A1 | 05-07-2018 |
| | | US 2019212160 A1 | 11-07-2019 |
| | | US 2022099452 A1 | 31-03-2022 |
| WO 2024159241 A2 | 02-08-2024 | NONE | |
| US 2023150551 A1 | 18-05-2023 | NONE | |

EPO FORM P0459

For more details about this annex : see Official Journal of the European Patent Office, No. 12/82